(19) Europäisches Patentamt
European Patent Office
Office européen des brevets

(11) Veröffentlichungsnummer: **0 273 862 B1**

(12) # EUROPÄISCHE PATENTSCHRIFT

(45) Veröffentlichungstag der Patentschrift: **10.07.91**

(51) Int. Cl.$^5$: **A01N 25/02**, A61K 47/00

(21) Anmeldenummer: **87810705.1**

(22) Anmeldetag: **30.11.87**

(54) **Ektoparasitizide nicht-wässrige Pour-on-Flüssigformulierung.**

(30) Priorität: **05.12.86 CH 4865/86**

(43) Veröffentlichungstag der Anmeldung:
**06.07.88 Patentblatt 88/27**

(45) Bekanntmachung des Hinweises auf die Patenterteilung:
**10.07.91 Patentblatt 91/28**

(84) Benannte Vertragsstaaten:
**AT BE CH DE ES FR GB GR IT LI LU NL SE**

(56) Entgegenhaltungen:
**EP-A- 0 136 033**
**AT-A- 9 187 582**
**FR-A- 1 495 304**

(73) Patentinhaber: **CIBA-GEIGY AG**
**Klybeckstrasse 141**
**CH-4002 Basel(CH)**

(72) Erfinder: **Metzner, Helmut**
**Im Jürtli 9**
**CH-4148 Pfeffingen(CH)**
Erfinder: **Steiner, Theodor, Dr.**
**Hinter Gärten 7**
**CH-4125 Riehen(CH)**
Erfinder: **Mayer, Peter, Dr.**
**Byfangweg 21**
**CH-4051 Basel(CH)**

**EP 0 273 862 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue nicht-wässrige flüssige Pour-on-Formulierungen gegen Ektoparasiten bei Nutztieren, die als charakteristisches Lösungsmittel Diacetonalkohol enthalten, welcher die Nachteile der in Pour-on-Formulierungen gebräuchlichen Lösungsmittel nicht aufweist. Die Erfindung betrifft ferner die Verwendung von Diacetonalkohol für die Herstellung von Flüssigformulierungen, welche gegen Ektoparasiten wirksam sind.

Eine ganze Reihe von Patentpublikationen, auf die nachstehend noch eingegangen wird, beschreibt Pour-on-Formulierungen und belegt gleichzeitig das grosse Interesse der Veterinärmedizin an dieser Applikationsform, die herkömmliche arbeitsintensive Behandlungsmethoden, wie z.B. Tauchbäder und Sprühgänge, durch einfache, zeit-und arbeitssparende Massnahmen zu ersetzen vermag.

Pour-on-Formulierungen zeichnen sich dadurch aus, dass der Wirkstoff in geeigneten flüssigen Medien, beispielsweise einem Lösungsmittel, gegebenenfalls unter Zugabe weiterer Hilfsstoffe, gelöst, emulgiert oder suspendiert wird und die resultierende flüssige Formulierung auf eine begrenzte Stelle der Körperoberfläche eines Nutztieres appliziert wird. Dies geschieht beispielsweise dadurch, dass man ein Tier auf dem Rücken oder einer anderen leicht zugänglichen Stelle einmalig mit der Flüssigformulierung betupft oder kurz ansprüht.

Die Vorteile einer solchen Behandlungsmethode liegen klar auf der Hand. Es sind dies beispielsweise:

1) Aeusserst einfache Handhabung.

2) Keine teuren Anlagen wie Tauchbäder und Sprühgänge notwendig.

3) Oftmals schwierige Wasser- und Energieversorgung der unter 2) genannten Anlagen entfällt.

4) Keine Probleme mit Einstellung gleichbleibender Anwendungskonzentrationen wie in Bädern - keine Strippingeffekte.

5) Die Tiere können an ihrem jeweiligen Aufenthaltsort behandelt werden und müssen nicht extra zu speziellen Applikationsanlagen getrieben oder transportiert werden - Ausschaltung von Transportrisiken.

Diese Vorteile von Pour-on-Applikationen sind dem Fachmann hinlänglich bekannt. Aus heutiger Sicht sollte eine Pour-on-Formulierung jedoch nicht nur für eine rasche Verteilung der Aktivsubstanz auf der Haut bzw. im Fell des Tieres sorgen, sondern als flüssiges Medium nur solche Lösungsmittel bzw. Formulierungshilfsstoffe enthalten, die für den Anwender und das behandelte Tier in jeder Hinsicht unbedenklich sind. Gerade das letzte Erfordernis wird von den meisten bekannten ektoparasitiziden Pour-on-Formulierungen nicht voll erfüllt, obgleich es sich bei den bekannten Formulierungen um sehr wirksame Mittel handeln kann.

GB-2,088,212 beschreibt eine Flüssig-Pour-on-Formulierung für die Anwendung bei Schafen, bestehend aus einem Pyrethroid, Xylol, Cyclohexanon und Pflanzenöl und eine Formulierung mit Diethylenglykol-mono-butylether als Hauptbestandteil, die noch Anteile von Isopropylmyristat enthalten kann.

Aus der Australischen Patentanmeldung Nr. 91875/82 sind flüssige Pour-on-Formulierungen auf der Basis von Glykolen, wie Ethylenglykol, Propylenglykol, etc., Glykolethern und Glykolestern bekannt.

Glykole und Glykolether mit $C_1$-$C_{10}$-Fettsäuren werden auch in der EP-120286 als Formulierungshilfsstoffe für Pour-on-Formulierungen vorgeschlagen.

Desgleichen nennt EP-61208 generell Glykole und insbesondere den Diethylenglykol-mono-butylether als Formulierungshilfsstoff für den entsprechenden Einsatz.

Dem Fachmann ist jedoch bekannt, dass die verschiedenen Nutztierarten in Bezug auf Pour-on-Lösungsmittel sehr unterschiedlich reagieren können. So werden z.B. Lösungsmittel wie Xylol und Cyclohexanon und Mischungen davon von Rindern in der Regel gut vertragen, während sie bei Schafen starke, nicht tolerierbare Reaktionen bewirken können, die zu Hautläsionen mit anschliessender Vernarbung führen [vgl. Britt A.G. et al., Australian Veterinary Journal, 61 (1984) 329]. Von Schafen gut toleriert werden andererseits Ethylenglykolether und Diethylenglykolether. Nachteilig an diesen Lösungsmitteln ist, dass sie auf Grund neuester Erkenntnisse nicht mehr als völlig unbedenklich eingestuft werden können und ihr Einsatz eher vermieden werden sollte.

Für die Verwendung beim Schaf wurde schon versucht (vgl. DE 3,238,525), nachteilige Lösungsmittel wie Xylol oder Cyclohexanon durch wässrige Vehikel zu ersetzen. Tatsächlich resultierten wässrige Formulierungen, die bezüglich Hautirritationen völlig unbedenklich sind. Diese an sich sehr positive Eigenschaft wird aber um den Preis einer wesentlich schlechteren Penetration ins Vliess und eines signifikant geringeren Selbstverteilungsvermögens auf dem Schaf erkauft, so dass insgesamt eine geringere Verlässlichkeit der Wirkung entsprechender Formulierungen resultiert, welche sich unmittelbar in einer kleineren Akzeptanz derartiger Produkte beim Verwender äussert.

Es versteht sich von selbst, dass die vielen Hydrolyse-empfindlichen Ektoparasitizide nicht in Form von wässrigen Systemen formuliert werden können.

Ferner fällt auf, dass der Grossteil der auf dem Markt erhältlichen ektoparasitiziden Pour-on-Formulierungen für Schafe als hautverträgliche Komponenten ausschliesslich Diethylenglykol-mono-butyl-ether aufweisen, eine Substanz, die, wie weiter oben aufgeführt, ersetzt werden sollte.

All dies zeigt, dass ein tatsächlicher Bedarf an einem Pour-on-Lösungsmittel besteht, das möglichst alle Anforderungen erfüllt, die an einen modernen Pour-on-Formulierungshilfsstoff gestellt werden, wie z.B. gute Penetration und Verteilung auf der Haut oder im Fell des Nutztieres, Hautverträglichkeit und Unbedenklichkeit, sowie eine universelle Anwendbarkeit bei unterschiedlichen Tierarten.

Es wurde nun gefunden, dass überraschenderweise Diacetonalkohol diese Eigenschaften besitzt und die oben erwähnten Forderungen erfüllt.

Diacetonalkohol ist chemisch gesehen 4-Hydroxy-4-methyl-pentan-2-on mit folgender Formel

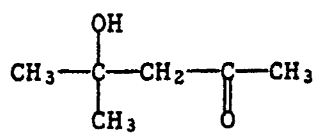

Diacetonalkohol siedet bei 164° C und wird gebildet aus Aceton durch Dimerisierung bei Behandlung mit basischen Reagenzien wie Bariumhydroxid.

Wasser-Diacetonalkohol-Gemische werden in dem Belgischen Patent Nr. 687,531 als Lösungsmittel für Pestizide, insbesondere für die Anwendung im Holzschutz vorgeschlagen. Ferner ist Diacetonalkohol als Lösungsmittel für Nagellacke bekannt.

Keine der vorgenannten Vorpublikationen enthält jedoch die geringste Andeutung, dass diese Substanz sich ausgezeichnet als Lösungsmittel für ektoparasitizide Wirksubstanzen in flüssigen Pour-on-Formulierungen eignet und im Rahmen dieser neuen Anwendung praktisch universell einsetzbar ist und die an ein modernes Pour-on-Lösungsmittel gestellten Anforderungen voll erfüllt. Mit seinen hervorragenden Lösungs- und Verteilungseigenschaften eignet es sich in hervorragender Weise für die Herstellung von ektoparasitiziden, nicht-wässrigen flüssigen Pour-on-Formulierungen auf der Basis von Wirkstoffen, welche ausgewählt sind aus der Gruppe Organophosphorverbindungen, Organochlorverbindungen, Pyrethroide, Carbamate, Amidine, Dimiloide, Triazine und Cyanopyrimidine sowie Gemischen dieser Wirkstoffe untereinander.

Zu den Dimiloiden, die in den erfindungsgemässen Formulierungen einsetzbar sind, zählen auch jene, die in WO 86/03941 oder der dort zitierten Literatur beschrieben sind, insbesondere jedoch die nachfolgend genannten Verbindungen der Formel X

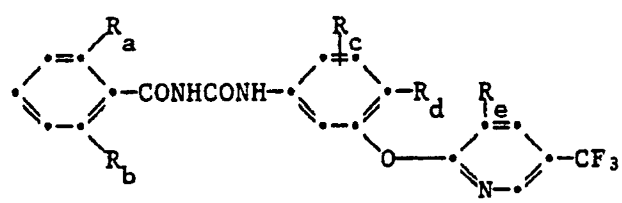 (X)

worin

- $R_a$ für Wasserstoff, $C_1$-$C_2$-Alkoxy, $C_1$-$C_2$-Alkylthio, $C_1$-$C_2$-Alkyl oder Halogen steht;
- $R_b$ Wasserstoff oder Halogen bedeutet;
- $R_c$ für Wasserstoff oder Halogen steht;
- $R_d$ Wasserstoff, $C_1$-$C_2$-Alkyl oder Halogen bedeutet; und
- $R_e$ für Halogen steht.

Bevorzugt werden Verbindungen der Formel X, worin

- $R_a$ für Fluor, Chlor, Methoxy, Methylthio oder Methyl steht;
- $R_b$ Wasserstoff, Fluor oder Chlor bedeutet;
- $R_c$ Wasserstoff oder in para-Stellung zur Pyridyloxygruppe befindliches Fluor oder Chlor bedeutet;
- $R_d$ Fluor, Chlor oder Brom bedeutet; und
- $R_e$ für Fluor oder Chlor steht.

Besonders bevorzugt werden Verbindungen der Formel X, worin

- $R_a$ für Fluor, Methoxy oder Methylthio steht;

$R_b$ für Fluor steht;
$R_c$ Wasserstoff bedeutet;
$R_d$ Chlor, Brom oder Methyl bedeutet; und
$R_e$ für Chlor steht.

Bevorzugte Einzelsubstanzen der Formel X sind u.a.:

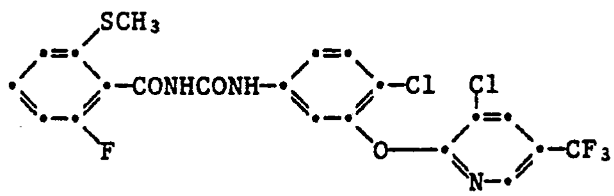
Smp. 193-195°C

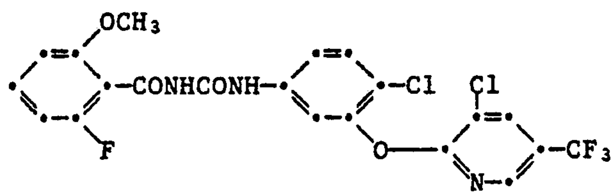
Smp. 180-182°C

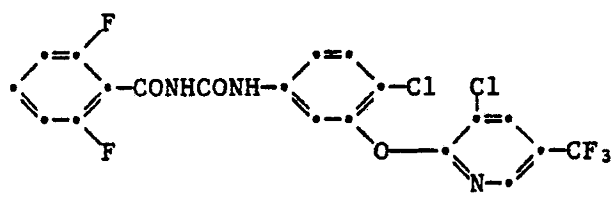
Smp. 185-189°C

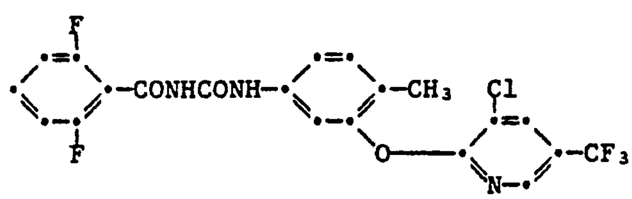
Smp. 178-179°C

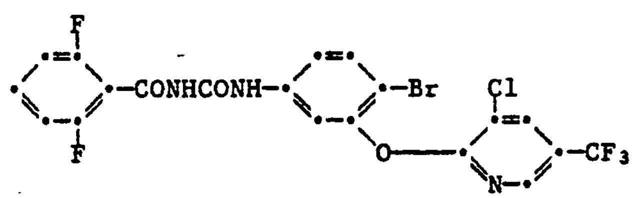
Smp. 198-200°C

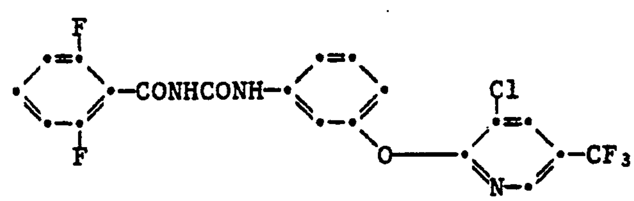
Smp. 172-173°C

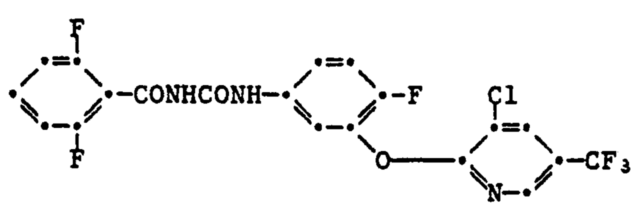
Smp. 211-212°C

Typische Vertreter von Verbindungen der Formel X sind:

| $R_a$ | $R_b$ | $R_c$ | $R_d$ | $R_e$ |
|---|---|---|---|---|
| H | H | 2-Cl | H | Cl |
| H | F | 2-Cl | Cl | Cl |
| F | F | H | Cl | Cl |
| F | Cl | 2-Cl | H | Cl |
| F | F | H | Br | Cl |
| F | F | H | $CH_3$ | Cl |
| F | F | H | $C_2H_5$ | Cl |
| $OCH_3$ | F | H | Cl | Cl |
| $OCH_3$ | F | H | $CH_3$ | Cl |
| $OCH_3$ | F | H | $C_2H_5$ | Cl |
| $OC_2H_5$ | F | H | Cl | Cl |
| $SCH_3$ | F | H | Cl | F |
| $SCH_3$ | F | H | $CH_3$ | Cl |
| $SCH_3$ | F | H | $C_2H_5$ | Cl |
| $SCH_3$ | F | H | Cl | Cl |
| $SC_2H_5$ | F | H | Cl | Cl |
| Cl | Cl | H | Cl | Cl |
| F | F | H | Cl | Br |
| F | F | 2-Cl | Cl | Cl |
| F | F | 3-Cl | Cl | Cl |
| F | F | 2-F | Cl | Cl |
| F | F | 2-Br | F | Cl |
| F | Br | H | Cl | Cl |

$R_c$ in 2-Position: in para-Position zur Pyridyloxygruppe; $R_c$ in 3-Position: direkt benachbart zu $R_d$.

Die Verbindungen der Formel X sind bekannt oder können analog bekannten Verfahren, wie sie beispielsweise in WO 86/03941 oder der darin zitierten Literatur beschrieben sind, hergestellt werden.

Bevorzugte Cyanopyrimidine, die als Ektoparasitizide in den erfindungsgemässen Pour-on-Flüssigformulierungen eingesetzt werden können, sind beispielsweise jene der nachstehenden Formel XX

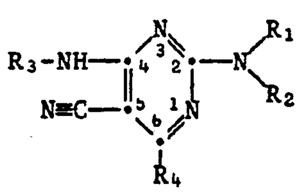

(XX),

worin

| | |
|---|---|
| $R_1$ | Wasserstoff, $C_1$-$C_6$-Alkyl, $C_2$-$C_6$-Alkenyl oder $C_2$-$C_6$-Alkinyl; |
| $R_2$ | Wasserstoff, $C_1$-$C_{10}$-Alkyl oder $C_3$-$C_6$-Cycloalkyl; oder |
| $R_1$ und $R_2$ | zusammen einen der Reste $-(CH_2)_3-$, $-(CH_2)_4-$ oder $-(CH_2)_5-$; |
| $R_3$ | Wasserstoff oder einen der Reste |

$-CO-R_5$ oder $-SO_2-R_6$ ;

$R_4$ einen der Reste

$-NH_2$, $-NH-CO-R_5$, $-NH-SO_2-R_6$ ,

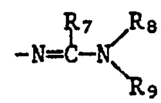

oder $-NH-CH=N-R_{10}$ ;

$R_5$ Wasserstoff, $C_1$-$C_{12}$-Alkyl, $C_3$-$C_6$-Cycloalkyl, $C_1$-$C_6$-Alkoxy, perhalogeniertes $C_1$-$C_3$-Alkyl oder den Rest

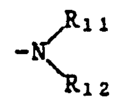 ;

$R_6$ $C_1$-$C_6$-Alkyl;

$R_7$ Wasserstoff oder $C_1$-$C_6$-Alkyl;

$R_8$ und $R_9$ unabhängig voneinander Wasserstoff oder $C_1$-$C_6$-Alkyl oder zusammen einen der Reste $-(CH_2)_3-$, $-(CH_2)_4-$ oder $-(CH_2)_5-$;

$R_{10}$ einen der Reste $-SO_2-R_{13}$ oder

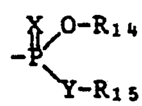 ;

$R_{11}$ und $R_{12}$ unabhängig voneinander Wasserstoff oder $C_1$-$C_6$-Alkyl oder zusammen einen der Reste $-(CH_2)_3-$, $-(CH_2)_4-$ oder $-(CH_2)_5-$;

$R_{13}$ $C_1$-$C_{10}$-Alkyl, mit bis zu 10 Halogenatom substituiertes $C_1$-$C_{10}$-Alkyl oder $C_3$-$C_6$-Cycloalkyl;

$R_{14}$ und $R_{15}$ unabhängig voneinander $C_1$-$C_{10}$-Alkyl;

X und Y unabhängig voneinander Sauerstoff oder Schwefel bedeuten, sowie deren Salze.

Unter Halogenatomen und Halogen-Substituenten im Rahmen der Formel XX sind Fluor, Chlor, Brom oder Jod, vorzugsweise Fluor und Chlor, zu verstehen.

Wegen ihrer vorteilhaften Wirkung sind solche Verbindungen der Formel XX bevorzugt, worin

$R_1$ Wasserstoff oder $C_1$-$C_4$-Alkyl;

$R_2$ $C_1$-$C_4$-Alkyl oder $C_3$-$C_6$-Cycloalkyl oder

$R_1$ und $R_2$ zusammen einen der Reste $-(CH_2)_4-$ oder $-(CH_2)_5-$;

$R_3$ Wasserstoff;

$R_4$ einen der Reste $-NH_2$ oder $-NH-CO-R_5$; und

$R_5$ $C_1$-$C_4$-Alkyl oder perhalogeniertes $C_1$-$C_3$-Alkyl bedeuten, und deren Salze, sowie solche Verbindungen der Formel I, worin

$R_1$ Wasserstoff, Methyl oder Aethyl;

$R_2$ $C_1$-$C_4$-Alkyl oder Cyclopropyl;

$R_3$ Wasserstoff;

$R_4$ einen der Reste $-NH_2$ oder $-NH-CO-R_5$;

$R_5$ $C_1$-$C_3$-Alkyl bedeuten, sowie deren Salze.

Aufgrund ihrer hohen pestiziden Aktivität sind insbesondere Verbindungen der Formel XX hervorzuheben, worin

$R_1$ Wasserstoff, Methyl oder Aethyl;

$R_2$ Cyclopropyl;

$R_3$ Wasserstoff; und

$R_4$ einen der Reste $-NH_2$ oder $-NH-CO-C_2H_5$ bedeuten, sowie deren Salze.

Unter Salzen der Verbindungen der Formel XX sind die physiologisch unbedenklichen Additionssalze anorganischer und organischer Säuren zu verstehen. Beispiele anorganischer Säuren sind Chlorwasserstoffsäure, Bromwasserstoffsäure, Jodwasserstoffsäure, Schwefelsäure, Phosphorsäure, phosphorige Säure und Salpetersäure. Beispiele organischer Säuren sind Trifluoressigsäure, Trichloressigsäure, Ameisensäure, Oxalsäure, Bernsteinsäure, Maleinsäure, Milchsäure, Glycolsäure, Aconitsäure, Zitronensäure, Benzoesäure, Benzolsulfonsäure und Methansulfonsäure.

Die Verbindungen der Formel XX können hergestellt werden, indem man eine Verbindung der Formel II

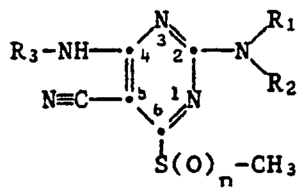

(II),

worin $R_1$, $R_2$ und $R_3$ die für Formel XX angegebenen Bedeutungen haben und n eine Zahl 0, 1 oder 2 bedeutet, mit Ammoniak umsetzt und die entstandene Verbindung der Formel XX, worin $R_4$ den Rest $-NH_2$ bedeutet, gegebenenfalls in an sich bekannter Weise in eine Verbindung der Formel XX überführt, worin $R_4$ die Bedeutungen $-NH-CO-R_5$, $-NH-SO_2-R_6$,

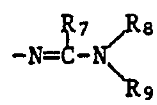

oder $-NH-CH=N-R_{10}$ hat, wobei $R_1$ bis $R_{15}$, X und Y die für Formel XX angegebenen Bedeutungen haben, und erwünschtenfalls eine erhaltene Verbindung der Formel XX in eines ihrer Salze überführt.

Die Umsetzung einer Sulfinyl- oder Sulfonylverbindung (n = 1 oder 2) der Formel II mit Ammoniak erfolgt allgemein bei einer Temperatur von etwa 10 bis 100 °C in organischen Lösungsmitteln, wie beispielsweise Acetonitril, Tetrahydrofuran, Dioxan oder Lösungsmittel-Wasser-Gemischen. Methylthioverbindungen (n = 0) der Formel II werden bevorzugt im Autoklaven unter erhöhtem Druck mit Ammoniak umgesetzt, und zwar bei einer Temperatur von 100 bis 160 °C, zumeist bei etwa 150 °C.

Die erwähnte Umwandlung einer Verbindung der Formel XX, worin $R_4$ $-NH_2$ bedeutet,in eine solche, worin $R_4$ eine andere der erfindungsgemäss angegebenen Bedeutungen hat, kann durch Umsetzung der in 6-Stellung am Pyrimidinring befindlichen Aminogruppe mit entsprechenden an sich bekannten Umsetzungsteilnehmern erfolgen. So kann man z.B. ein 6-Aminopyrimidin der Formel XX mit Verbindungen vom Typ $Hal-CO-R_5$, $Hal-SO_2-R_6$, $(R_5-CO)_2O$ oder $R_6O-CH=N-R_{10}$ umsetzen, um zu in 6-Stellung entsprechend substituierten Verbindungen der Formel XX zu gelangen, wobei Hal ein Halogenatom, vorzugsweise Chlor, darstellt und $R_5$, $R_6$ und $R_{10}$ die vorstehend angegebenen Bedeutungen haben. Wenn eine Verbindung der Formel XX hergestellt werden soll, worin $R_4$ den Rest

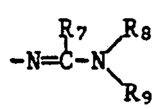

bedeutet, wird die 6-$NH_2$-Gruppe beisielsweise mit einem Acetal der Formel

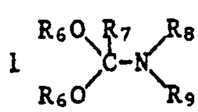

umgesetzt, wobei $R_6$, $R_7$, $R_8$ und $R_9$ die vorstehend angegebenen Bedeutungen haben. In analoger Weise und mit entsprechenden Umsetzungsteilnehmern kann man aus 4-Aminopyrimidinen der Formel XX, worin $R_3$ Wasserstoff bedeutet, Verbindungen der Formel XX herstellen, worin $R_3$ einen der Reste $-CO-R_5$ oder $-SO_2-R_6$ darstellt. Die vorstehend beschriebenen Acylierungen werden unter normalem Druck in inerten Lösungs- oder Verdünnungsmitteln in Gegenwart einer Base bei Temperaturen von 0 bis 120°C, vorzugsweise 40 bis 80°C, durchgeführt. Als Lösungs- und Verdünnungsmittel kommen beispielsweise in Frage: Alkane, vorzugsweise solche mit 1 bis 17 Kohlenstoffatomen, wie n-Pentan sowie seine Homologen einschliesslich der Isomeren bis zum n-Heptadecan; Aether wie Diäthyläther, Dipropyläther, Dibutyläther, Dimethoxyäthan, Dioxan oder Tetrahydrofuran; Chlorierte Kohlenwasserstoffe, wie Chloroform, Methylenchlorid, Tetrachlorkohlenstoff oder Chlorbenzol; aromatische Kohlenwasserstoffe wie Benzol, Toluol oder Xylole. Darüberhinaus können weitere inerte Lösungs- und Verdünnungsmittel bei dem Verfahren Anwendung finden. Als gegebenenfalls bei diesen Umsetzungen zu verwendende Basen eignen sich beispielsweise Alkylamine, wie Triäthylamin oder Diisopropyläthylamin, sowie ferner Pyridin oder N-Methylpyrrolidon.

Zu Verbindungen der Formel II kann man, wie nachstehend schematisch angegeben, folgendermassen gelangen:

a)

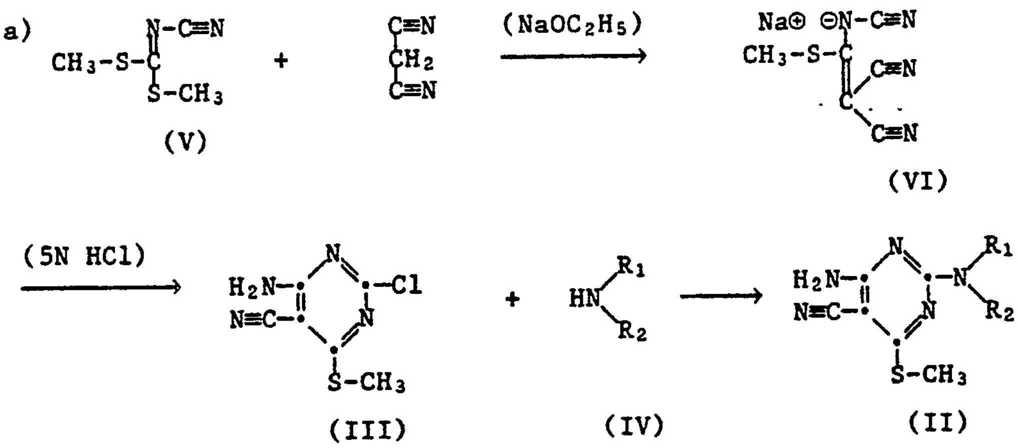


(V) (VI) (III) (IV) (II)

Der für das obige Verfahren als Ausgangsprodukt verwendete N-Cyanodithiocarbonimidsäuredimethylester der Formel V und das durch Umsetzung mit Malononitril entstehende Salz der Formel VI sind bekannt (vgl. Rec.Trav. Ch. 90/1971, 463; J.Chem.Soc., Chem. Comm. 1974, 350). Aus dem Salz der Formel VI kann man durch Ringschluss mit 5N HCl und anschliessende Umsetzung des gebildeten Pyrimidins der Formel III mit einen entsprechenden Amin der Formel IV eine Verbindung der Formel II erhalten, in welcher $R_3$ = Wasserstoff und n = 0 ist.

b)

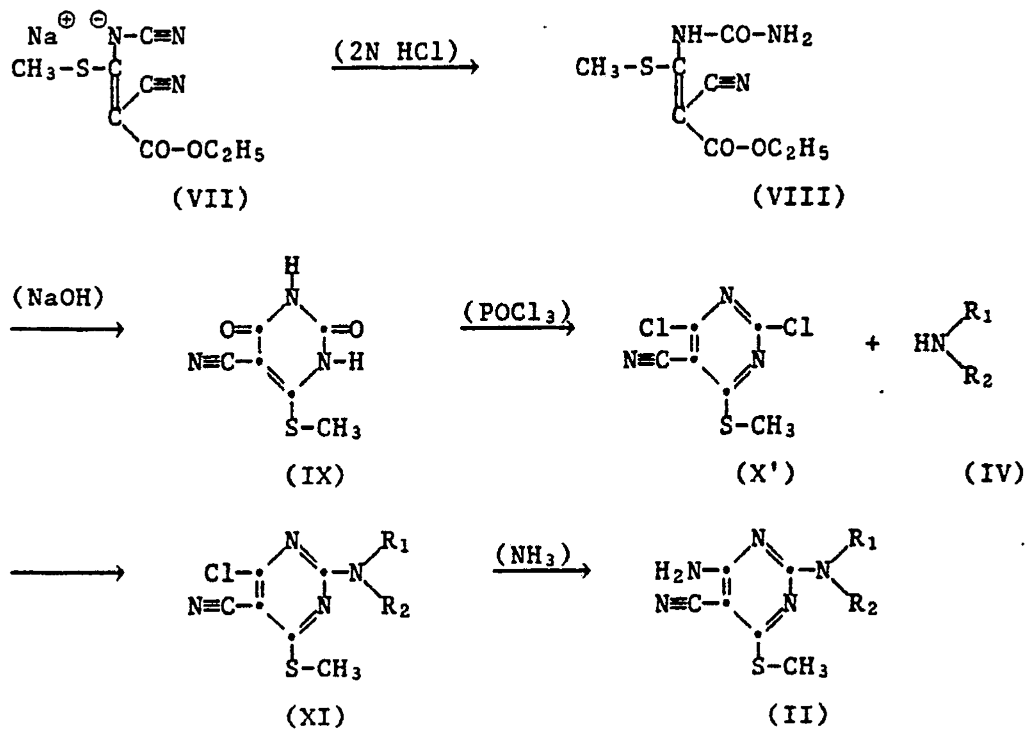


(VII) (VIII) (IX) (X') (IV) (XI) (II)

Das im obigen Reaktionsschema angegebene Natriumsalz der Formel VII, der Harnstoff der Formel VIII sowie das Uracil der Formel IX sind bekannt (vgl. J.Chem.Soc., Chem. Comm. 1974, 350; Helv. Chim. Act. 68, 1985, 1155). Die Herstellung der neuen substituierten Pyrimidine der Formeln X' und XI erfolgt in an sich bekannter Weise. Durch Umsetzung von XI mit Ammoniak wird eine Verbindung der Formel II erhalten, worin $R_3$ = Wasserstoff und n = 0 ist.

c) Verbindungen der Formel II, worin n = 2 ist können in an sich bekannter Weise durch Oxydation von Verbindungen der Formel II, worin n = 0 ist, hergestellt werden (vgl. "The Chemistry of Heterocyclic Compounds", Vol. 16: Pyrimidines, Intersc. Publ. Inc., N.Y. 1959). Auf analoge Weise lassen sich auch Verbindungen der Formel II herstellen, in denen n = 1 ist.

Nichtlimitierende Beispiele für ektoparasitizide Wirksubstanzen, die in den erfindungsgemässen nichtwässrigen Pour-on-Flüssigformulierungen eingesetzt werden können sind:

AMITRAZ = N,N-bis-(2,4-Xylyliminomethyl)methylamin;
PROPETAMPHOS = 1-Methylethyl-(E)-3-[[(ethyl-amino)methoxyphosphinothioyl]-oxy]-2-butensäureester;
CHLORPYRIFOS = Thiophosphorsäure-O,O-diethyl-O-(3,5,6-trichloro-2-pyridinyl)ester;
DIAZINON = Thiophosphorsäure-O,O-diethyl-O-[6-methyl-2-(1-methylethyl)-4-pyrimidinyl]ester;
COUMAPHOS = Thiophosphorsäure-O-(3-chlor-4-methyl-2-oxo-2H-l-benzopyran-7-yl)-O,O-diethylester;
MALATHION = [(Dimethoxyphosphinothioyl)thio]-bernsteinsäurediethylester;
CHLORFENVINPHOS = Phosphorsäure-2-chlor-l-(2,4-dichlorphenyl)-ethenyl-diethylester;
LINDANE = 1α, 2α, 3β, 4α, 5α, 6β-Hexachlorcyclohexan;
TIFATOL = N-(2,3-Dihydro-3-methyl-1,3-thiazol-2-yliden)-2,4-xylidin;
PHOXIM = O-(α-Cyanobenzylidenamino)-O'-O''-diethylthiophosphat;
FLUMETHRIN = 3-[2-(4-Chlorphenyl)-2-chlorvinyl]-2,2-dimethylcyclopropancarbonsäure-(α-cyano-4-fluor-3-phenoxy-benzylester;
CYROMAZIN = 2-Cyclopropylamino-4,6-diamino-s-triazin;
CYHALOTHRIN = (R,S)-α-Cyano-3-phenoxybenzyl(Z)-(1RS,3RS)-(2-chlor-3,3,3-trifluorpropenyl)-2,2-dimethylcyclopropancarboxylat;
DELTAMETHRIN = (S)-α-Cyano-3-phenoxybenzyl-(1R,3R)-3-(2,2-dibromovinyl)-2,2-dimethylcyclopropancarboxylat;
FENVALERAT = (RS)-α-Cyano-3-phenoxybenzyl-(RS)-2-(4-chlorphenyl)-3-methylbutyrat;
PERMETHRIN = 3-Phenoxybenzyl-(1RS)-cis,trans-3-(2,2-dichlorvinyl)-2,2-dimethylcyclopropancarboxylat;
CYPERMETHRIN = (RS)-α-cyano-3-phenoxybenzyl-(1RS)-cis,trans-3-(2,2-dichlorvinyl)-2,2-dimethylcyclopropanecarboxylat;

FAMPHUR = O-[4-(Dimethylsulfamoyl)-phenyl]-O,O-dimethylphosphorthioat;
FENTHION = O,O-Dimethyl-O-4-methylthio-m-tolylphosphorthioat;
PHOSMET = O,O-Dimethyl-S-phthalimidomethylphosphorodithioat;
TETRACHLORVINPHOS = [(Z)-2-Chlor-1-(2,4,5-trichlorphenyl)-vinyl]-dimethylphosphat;
PROMACYL = 5-Methyl-m-cumenylbutyryl(methyl)carbamat; und
DIFLUBENZURON = 1-(4-Chlorphenyl)-3-(2,6-difluorbenzoyl)-harnstoff.

Die Verwendung von Diacetonalkohol als Formulierungsbestandteil in flüssigen nicht-wässrigen Pour-on-Formulierungen von Ektoparasitiziden, vorzugsweise solchen der oben genannten Wirkstoffklassen, stellt einen wesentlichen Bestandteil der vorliegenden Erfindung dar.

Es hat sich erstaunlicherweise gezeigt, dass die erfindungsgemässen Pour-on-Formulierungen breit anwendbar sind, beispielsweise bei Schafen und Ziegen zur Bekämpfung von Läusen, Milben, Zecken, Fliegen, Schaflausfliegen, parasitierenden Fliegenlarven und anderen Ektoparasiten, sowie gegen Läuse, Milben, Zecken, Fliegen und weitere Ektoparasiten bei Rindern, Büffeln, Pferden und Kamelen, aber auch gegen Milben, Läuse und sonstige Ektoparasiten bei Schweinen.

Die vorliegende Erfindung betrifft somit im wesentlichen eine nicht-wässrige Pour-on-Flüssigformulierung von ektoparasitiziden Wirkstoffen zur Verwendung am Nutztier, die sich dadurch kennzeichnet, dass sie neben dem Wirkstoff oder Wirkstoffgemisch als Hauptkomponente Diacetonalkohol und gegebenenfalls als Nebenkomponente einen oder mehrere zweckdienliche, andere Formulierungshilfsstoffe enthält.

Der Ausdruck "Hauptkomponente" soll in diesem Zusammenhang klarstellen, dass der Gehalt an Diacetonalkohol in den erfindungsgemässen Formulierungen stets ein Vielfaches des Gehaltes an anderen Formulierungshilfsstoffen beträgt. Bevorzugt sind jene Formulierungen, die 0,1 bis 60 Gewichtsprozent eines ektoparasitiziden Wirkstoffs oder Wirkstoffgemisches, 40 bis 99,9 Gewichtsprozent an Diacetonalkohol und 0 bis 20 Gewichtsprozent andere Formulierungshilfsstoffe aufweisen. Als andere Formulierungshilfsstoffe gelten im Rahmen vorliegender Erfindung beispielsweise Cosolventien, Stabilisatoren, Farbstoffe, Antioxidantien, oberflächenaktive Verbindungen, Haftvermittler oder sonstige für topische Applikationsformen geeignete, toxisch unbedenkliche Formulierungshilfssubstanzen.

Geeignete Cosolventien sind beispielsweise niedere Alkohole, wie Ethanol, Propanol, iso-Propanol, Butanol oder Amylalkohol; mehrwertige Alkohole wie Glycerin; Ester, wie Essigsäureethylester, Propionsäuremethylester oder Essigsäurepropylester; alkylierte Amide, wie N,N-Dimethylacetamid oder N-Methylpyrrolidon; Dimethylsulfoxid; Ketone wie Methylethylketon, synthetische und natürliche Oele, wie aliphatische und aromatische Kohlenwasserstoffe und deren Gemische, Sojabohnenöl, Baumwollsaatöl, Kokosnussöl, Erdnussöl, Olivenöl, Maiskeimöl, Sesamöl oder Ricinusöl, wobei wenn besagte Oele epoxidierbar sind, deren Epoxide für viele ektoparasitizide Wirkstoffe auch die Funktion eines Stabilisators haben.

Als Haftvermittler können beispielsweise verschiedene Cellulose- und Stärke-Derivate, wie Carboxymethylcellulose oder Methylcellulose, Polyacrylsäureester, Alginate, Gelatine, Gummi-arabicum, Polyvinylpyrrolidon, Polyvinylalkohole, Paraffine, Wachse und Harze eingesetzt werden.

Als oberflächenaktive Verbindungen kommen je nach der Art des zu formulierenden Wirkstoffes nichtionogene, kation-und/oder anionaktive Tenside mit guten Emulgier, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Die in der Formulierungstechnik gebräuchlichen Tenside sind u.a. in folgender Publikation beschrieben:
"Mc Cutcheon's Detergents and Emulsifiers Annual"
MC Publishing Corp., Ridgewood, New Jersey, 1982.

Farbstoffe werden im Rahmen vorliegender Erfindung nur insofern als Formulierungshilfsstoffe bezeichnet, als sie als Bestandteile der Formulierung der optischen Unterscheidbarkeit von behandelten und unbehandelten Tieren dienen.

Die Mittel können auch weitere Zusätze wie Entschäumer, Viskositätsregulatoren, Bindemittel oder andere Wirkstoffe zur Erzielung spezieller zusätzlicher Effekte enthalten.

Die vorliegende Erfindung betrifft zwar nicht-wässrige Formulierungen, diese können jedoch zu Verdünnungszwecken, beispielsweise unmittelbar vor der Applikation auf das Tier, mit Wasser verdünnt werden.

Die erfindungsgemässen nicht-wässrigen Pour-on-Flüssigformulierungen werden erfindungsgemäss hergestellt, indem man den ektoparasitiziden Wirkstoff, vorzugsweise in einer fein gemahlenen Form, in Diacetonalkohol löst oder gegebenenfalls unter Zusatz von oberflächenaktiven Verbindungen emulgiert oder suspendiert und, falls erforderlich, die übrigen Hilfsingredienzien vorlegt oder der Mischung zusetzt.

Die nachfolgenden Beispiele dienen zur Illustration der Erfindung, haben jedoch keinen limitierenden Charakter. Die Prozentangaben in den Beispielen sind Gewichtsprozente.

Herstellungsbeispiele für die eingangs genannten Cyanopyrimidine

Beispiel 1

a) Herstellung der Ausgangsverbindung 2-Cyclopropylamino-4-amino-5-cyano-6-methylthio-pyrimidin:

i) Zu einer Lösung von 93 g 2-Cyano-3-cyanoamino-3-methylthioacrylnitril-Natriumsalz in 425 ml Wasser werden 650 ml konzentrierte Salzsäure unter Kühlung mittels Eiswasser zugetropft. Dann wird der Ansatz während ca. 12 Stunden bei Raumtemperatur gerührt. Der entstandene Niederschlag wir abfiltriert und mit wässriger Sodalösung behandelt. Das so erhaltene 2-Chlor-4-amino-5-cyano-6-methylthio-pyrimidin (Smp.: 268°C) wird in einer Menge von 20 g mit 250 ml Acetonitril zu einer Suspension verrührt, in welche unter kräftigem Rühren 11,4 g Cyclopropylamin bei Rückflusstemperatur eingetropft werden. Der Ansatz wird etwa 12 Stunden weiter gerührt und dann mit Wasser versetzt. Der gebildete Niederschalg wird abfiltriert. Man erhält so die Titelverbindung der Formel

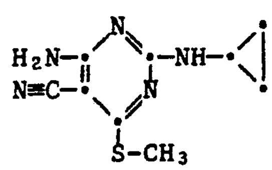

mit einem Smp. von 215-218°C (Verbindung Nr. 3.1.).

ii) Zu einem Gemisch aus 18,3 g 5-Cyano-6-methylthiouracil und 80 ml Phosphoroxychlorid werden 20 g N,N-Diäthylanilin langsam zugetropft. Der Ansatz wird dann während 1,5 Stunden am Rückfluss erhitzt und nachfolgend eingedampft. Der Rückstand wird mit 200 ml Eiswasser versetzt, verrührt, abfiltriert und mit Wasser gewaschen. Nach dem Trocknen wird das Rohprodukt an Kieselgel chromatographiert (Toluol/Chloroform/Essigester 6:3:1). 22 g des so erhaltenen 2,4-Dichlor-5-cyano-6-methylthio-pyrimidins (Smp. 118-120°C) werden in 200 ml Acetonitril gelöst, und bei -10°C wird eine Lösung von 11,4 g Cyclopropylamin in 40 ml Acetonitril zugetropft. Das Reaktionsgemisch wird bei Raumtemperatur während 2 Stunden gerührt und dann auf 2 ℓ Eiswasser gegossen. Der gebildete Rückstand wird abgesaugt. Das nach obiger Arbeitsweise erhaltene 2-Cyclopropylamino-4-chlor-5-cyano-6-methylthiopyrimidin der Formel

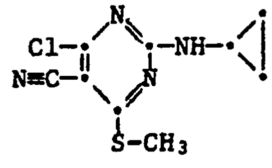

mit einem Smp. von 139-141 °C wird in einer Menge von 26,1 g mit 100 ml Acetonitril verrührt. Es werden 300 ml 30 %-iges wässriges Ammoniak zu der so entstandenen Suspension hinzugefügt, die dann etwa 10 Stunden bei Raumtemperatur und anschliessend 5 Stunden am Rückfluss gerührt wird. Der nach dem Abkühlen des Ansatzes entstandene feste Rückstand wird abgesaugt und mit Wasser gewaschen. Nach dem Umkristallisieren aus 150 ml 2-Methoxyäthanol (Methylcellosolve®) wird die Titelverbindung mit der unter i) angegebenen Formel von Smp. 215-218 °C erhalten.

b) Herstellung von 2-Cyclopropylamino-4,6-diamino-5-cyano-pyrimidin:

Es werden 33,7 g des gemäss a) hergestellten 2-Cyclopropylamino-4-amino-5-cyano-6-methylthiopyrimidins im Autoklaven bei 150 °C während 15-20 Stunden mit 150 g Ammoniak behandelt. Das gebildete Reaktionsprodukt wird mehrmals mit Wasser verrührt und dann abfiltriert. Man erhält auf diese Weise die Titelverbindung der Formel

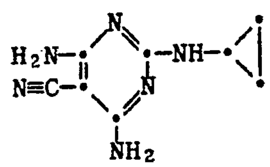

mit einem Smp. von 249-251 °C (Verbindung Nr. 1.1).

Beispiel 2

a) Herstellung der Ausgangsverbindung 2-Diäthylamino-4-amino-5-cyano-6-methylsulfonyl-pyrimidin:

Es werden 47,5 g 2-Diäthylamino-4-amino-5-cyano-6-methylthiopyrimidin in 650 ml Methylenchlorid vorgelegt. Zu dem Ansatz werden 88,7 g 3-Chlorperbenzoesäure ohne Kühlung hinzugefügt. Das Reaktionsgemisch wird während 2 Stunden gerührt und dann filtriert. Das Filtrat wird eingedampft, der Rückstand mit Diäthyläther aufgeschlämmt und abgesaugt. Man erhält die Titelverbindung der Formel

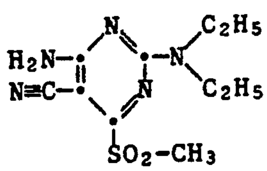

mit einem Smp. von 170-172 °C (Verbindung Nr. 3.2).

b) Herstellung von 2-Diäthylamino-4,6-diamino-5-cyano-pyrimidin:

Zu einem aus 300 ml 30 %-igem wässrigen Ammoniak und 100 ml Acetonitril bestehenden Gemisch werden bei Raumtemperatur 26,9 g des gemäss a) hergestellten 2-Diäthylamino-4-amino-5-cyano-6-methylsulfonyl-pyrimidins eingetragen. Der Ansatz wird dann etwa 12 Stunden am Rückfluss gehalten, abgekühlt und von dem gebildeten Niederschlag abfiltriert. Der Niederschlag wird mit Wasser behandelt und abgesaugt. Man erhält so die Titelverbindung der Formel

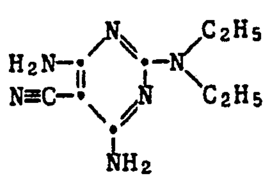

mit einem Smp. von 222-224°C (Verbindung Nr. 1.2).

Beispiel 3

Herstellung von 2-Diäthylamino-4-amino-5-cyano-6-isobutyrylaminopyrimidin:

Zu einer Lösung von 10,3 g 2-Diäthylamino-4,6-diamino-5-cyanopyrimidin (hergestellt nach Beispiel 2) in 90 ml Tetrahydrofuran werden 7 g Triäthylamin hinzugesetzt. In diese Lösung werden bei 60°C 8,5 g Isobuttersäureanhyrid eingetropft. Das Reaktionsgemisch wird während 48 Stunden am Rückfluss erhitzt, eingedampft und der gebildete Rückstand mit Diäthyläther gewaschen. Die so erhaltene Titelverbindung der Formel

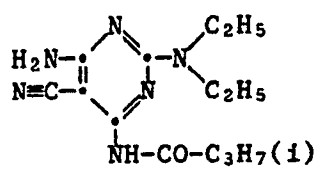

hat einem Smp. von 151-152°C (Verbindung Nr. 1.3).

Beispiel 4:

Herstellung von N,N-Dimethyl-N'-(2-cyclopropylamino-4-amino-5-cyano-pyrimidin-6-yl)-formamidin:

Es werden 3,7 g Dimethylformamiddiäthylacetal zu einer Lösung von 3,8 g 2-Cyclopropylamino-4,6-diamino-5-cyanopyrimidin in 100 ml Dioxan bei 45°-50°C zugetropft. Das Reaktionsgemisch wird während 8 Stunden bei 45 bis 50°C gerührt. Nach dem Abdestillieren des Lösungsmittels und Umkristallisieren des Rohproduktes aus Aethanol wird die Titelverbindung der Formel

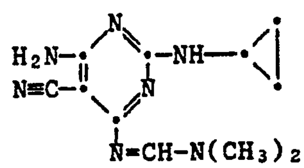

mit einem Smp. von 192-195°C erhalten (Verbindung Nr. 1.4).

Analog den vorstehend aufgezeigten Arbeitsweisen werden auch die folgenden Verbindungen der Formel XX hergestellt und können als Wirkstoffe in den erfindungsgemässen Formulierungen eingesetzt werden:

| Verb. Nr. | $R_1$ | $R_2$ | $R_3$ | $R_4$ | Smp. [°C] |
|---|---|---|---|---|---|
| 1.5 | H | $-C_3H_7(i)$ | H | $-NH_2$ | 230-232 |
| 1.6 | H | $-CH(CH_3)-C_2H_5$ | H | $-NH_2$ | 195-197 |
| 1.7 | $-CH_3$ | $-CH_3$ | H | $-NH_2$ | > 260 |
| 1.8 | $-C_3H_7(n)$ | $-C_3H_7(n)$ | H | $-NH_2$ | 204-206 |
| 1.9 | $-C_4H_9(n)$ | $-C_4H_9(n)$ | H | $-NH_2$ | 182-185 |
| 1.10 | $-CH_3$ | $-C_2H_5$ | H | $-NH_2$ | 250-251 |
| 1.11 | $-(CH_2)_4-$ | | H | $-NH_2$ | > 260 |
| 1.12 | $-(CH_2)_5$ | | H | $-NH_2$ | 234-236 |
| 1.13 | H | $-C(CH_3)_3$ | H | $-NH_2$ | 180-183 |
| 1.14 | $-CH_3$ | $-C_4H_9(n)$ | H | $-NH_2$ | 194-196 |
| 1.15 | H | 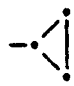 | H | $-NH-CO-CH(CH_3)_2$ | 215-218 |
| 1.16 | H | 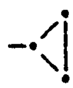 | H | $-NH-CO-C_2H_5$ | 180-181 |
| 1.17 | H | 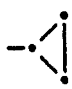 | H | $-NH-CO-CH_3$ | 203-205 |
| 1.18 | $-C_2H_5$ | $-C_2H_5$ | H | $-NH-CO-C_2H_5$ | 158-160 |
| 1.19 | $-C_2H_5$ | $-C_2H_5$ | H | $-NH-CO-C(CH_3)_3$ | 113-115 |
| 1.20 | $-C_2H_5$ | $-C_2H_5$ | H | $-NH-CO-CH_3$ | 168-171 |
| 1.21 | $-C_2H_5$ | $-C_2H_5$ | H | $-NH-CO-C_3H_7(n)$ | 149-150 |
| 1.22 | H | 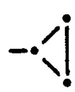 | H | $-NH-CO-C(CH_3)_3$ | 110-113 |
| 1.23 | $-C_2H_5$ | $-C_2H_5$ | H | $-NH-CO-C_4H_9(n)$ | 119-120 |
| 1.24 | H | 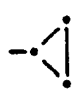 | H | $-NH-CO-C_3H_7(n)$ | 175-176 |

| Verb. Nr. | $R_1$ | $R_2$ | $R_3$ | $R_4$ | Smp. [°C] |
|---|---|---|---|---|---|
| 1.25 | H | 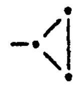 | H | $-NH-CO-C_4H_9(n)$ | 187-188 |
| 1.26 | $-C_2H_5$ | $-C_2H_5$ | H | $-NH-CO-CF_3$ | 190-191 |
| 1.27 | H | 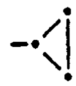 | H | $-NH-CO-CF_3$ | 268 |
| 1.28 | $-CH_3$ | $-C_4H_9(n)$ | H | $-NH-CO-CH(CH_3)_2$ | 139-140 |
| 1.29 | $-CH_3$ | $-C_2H_5$ | H | $-NH-CO-CH(CH_3)_2$ | 146-149 |
| 1.30 | H | 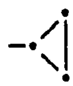 | H | $-NH-CO-CH(C_2H_5)_2$ | 196-197 |
| 1.31 | $-C_2H_5$ | $-C_2H_5$ | H | $-NH-CO-(CH_2)_4CH_3$ | 133-135 |
| 1.32 | H | 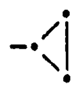 | H | $-NH-CO-(CH_2)_4CH_3$ | 170-173 |
| 1.33 | H | 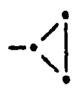 | H | $-N=CH-N(CH_3)-CH_3$ | 192-195 |
| 1.34 | $-C_2H_5$ | $-C_2H_5$ | H | $-N=CH-N(CH_3)-CH_3$ | 160-165 |
| 1.35 | H | 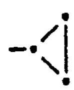 | H | $-NH-CH=N-SO_2CH_3$ | 203-205 |
| 1.36 | $-C_2H_5$ | $-C_2H_5$ | H | $-NH-CH=N-SO_2CH_3$ | 182-185 |
| 1.37 | $-C_2H_5$ | $-C_2H_5$ | H | $-NH-CH=N-P(=O)(OC_2H_5)SC_3H_7(n)$ | 127-128 |

Die folgenden Salze von Verbindungen der Formel XX werden durch Umsetzung mit den jeweils bezeichneten Säuren hergestellt und sind erfindungsgemäss einsetzbar.

| Verb. Nr. | $R_1$ | $R_2$ | $R_3$ | $R_4$ | Säure | Smp. [°C] |
|---|---|---|---|---|---|---|
| 2.1 | H | 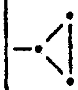 | $-NH_2$ | $-NH_2$ | HCl | 210 (Zers.) |
| 2.2 | H | 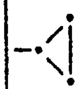 | $-NH_2$ | $-NH_2$ | $F_3C-COOH$ | 209-210 |
| 2.3 | H | 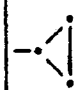 | $-NH_2$ | $-NH_2$ | $(COOH)_2$ | 198 (Zers.) |
| 2.4 | $-C_2H_5$ | $-C_2H_5$ | $-NH_2$ | $-NH_2$ | HCl | 167-169 |
| 2.5 | $-C_2H_5$ | $-C_2H_5$ | $-NH_2$ | $-NH_2$ | $F_3C-COOH$ | 175-178 |
| 2.6 | $-C_2H_5$ | $-C_2H_5$ | $-NH_2$ | $-NH_2$ | $(COOH)_2$ | 178-188 |
| 2.7 | H | 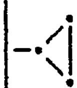 | $-NH_2$ | $-NH-CO-CH(CH_3)_2$ | HCl | ~140 |
| 2.8 | H | 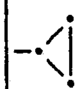 | $-NH_2$ | $-NH-CO-CH(CH_3)_2$ | $CF_3COOH$ | 150-153 |
| 2.9 | H | 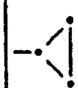 | $-NH_2$ | $-NH_2$ | $H_3PO_4$ | 208 |
| 2.10 | H | 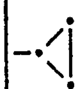 | $-NH_2$ | $-NH_2$ | $H_2SO_4$ | 188 |
| 2.11 | $-C_2H_5$ | $-C_2H_5$ | $-NH_2$ | $-NH_2$ | $H_2SO_4$ | 199-201 |
| 2.12 | $-C_2H_5$ | $-C_2H_5$ | $-NH_2$ | $-NH_2$ | $H_3PO_4$ | 185 |
| 2.13 | $-C_2H_5$ | $-C_2H_5$ | $-NH_2$ | $-NH_2$ | $CH_3SO_3H$ | 205-207 |
| 2.14 | H | 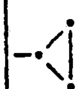 | $-NH_2$ | $-NH_2$ | $CH_3SO_3H$ | 250 |
| 2.15 | $-C_2H_5$ | $-C_2H_5$ | $-NH_2$ | $-NH_2$ | Maleinsäure | 150 |
| 2.16 | H | 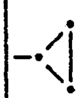 | $-NH_2$ | $-NH_2$ | Maleinsäure | 185 |
| 2.17 | $-C_2H_5$ | $-C_2H_5$ | $-NH_2$ | $-NH_2$ | Malonsäure | 216-220 |

| Verb. Nr. | $R_1$ | $R_2$ | $R_3$ | $R_4$ | Säure | Smp. [°C] |
|---|---|---|---|---|---|---|
| 2.18 | H | 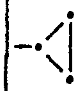 | $-NH_2$ | $-NH_2$ | $HNO_3$ | 163 |
| 2.19 | $-C_2H_5$ | $-C_2H_5$ | $-NH_2$ | $-NH_2$ | $HNO_3$ | 179 |
| 2.20 | H | 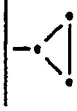 | $-NH_2$ | $-NH_2$ | Malonsäure | >230 |

Wie vorstehend angegeben sind auch die folgenden Verbindungen der Formel XX herstellbar und sind erfindungsgemäss einsetzbar:

| $R_1$ | $R_2$ | $R_3$ | $R_4$ |
|---|---|---|---|
| H | $-CH_3$ | H | $-NH_2$ |
| H | $-C_2H_5$ | H | $-NH_2$ |
| H | $-C_3H_7(n)$ | H | $-NH_2$ |
| H | 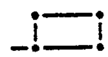 | H | $-NH_2$ |
| H | 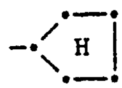 | H | $-NH_2$ |
| H | 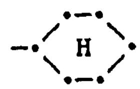 | H | $-NH_2$ |
| H | $-CH(C_2H_5)_2$ | H | $-NH_2$ |
| $-C_3H_7(i)$ | $-C_3H_7(i)$ | H | $-NH_2$ |
| H | 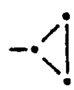 | H | 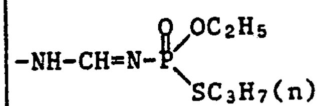 |
| H | 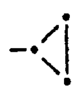 | H | $-NH-CO-C_2H_5$ |
| $-C_2H_5$ | $-C_2H_5$ | H | $-NH-CO-C_2H_5$ |
| $-C_2H_5$ | $-C_2H_5$ | H | $-NH-CO-CH_2Cl$ |
| H | 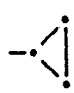 | H | $-NH-CO-C_2F_5$ |
| $-C_2H_5$ | $-C_2H_5$ | H | $-NH-CO-C_2F_5$ |
| H | 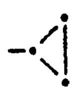 | H | $-NH-CO-C_3F_7(n)$ |
| $-C_2H_5$ | $-C_2H_5$ | H | $-NH-CO-C_3F_7(n)$ |

Beispiele erfindungsgemässer Formulierungen:

**Beispiel F1:**

| | |
|---|---|
| Cypermethrin (high cis)<br>= cis-3-(2,2-dichlorovinyl)-2,2-dimethyl cyclopropancarbonsäure-α-cyano-3-phenoxybenzylester | 2 % |
| Diacetonalkohol | 98 % |

Im Rahmen der vorliegenden Erfindung sind Formulierungen mit den Wirkstoffen Cypermethrin (high

cis und cis/trans) und Diazinon besonders bevorzugt.

Beispiel F2:

| | |
|---|---|
| Cypermethrin (high cis) | 2 % |
| C.I. Acid Blue 80 (mit C.I. Nr. 61585) | 0,1 % |
| Diacetonalkohol | 97,9 % |

Beispiel F3:

| | |
|---|---|
| Diazinon = Thiophosphorsäure-0,0-diethyl-0-[6-methyl-2-(1-methylethyl-4-pyrimidinyl]-ester | 10 % |
| Epoxidiertes Soyabohnenöl | 5 % |
| Diacetonalkohol | 85 % |

Beispiel F4:

| | |
|---|---|
| Diazinon | 50 % |
| Epoxidiertes Sojabohnenöl | 15 % |
| Diacetonalkohol | 35 % |

Biologische Beispiele:

Beispiel B1: Anwendung gegen Schaflaus

Natürlich und stark mit Damalinia ovis (Schaflaus) infizierte weibliche Schafe der Kreuzung Romney Marsh in Neusseland werden mit der Formulierung gemäss Beispiel F1 durch Aufguss entlang der Rückenlinie behandelt. Vor der Applikation wird sichergestellt, dass alle Stadien des Entwicklungszyklus der Schaflaus vorhanden sind. Unbehandelte Schafe werden getrennt unter den gleichen Bedingungen gehalten und dienen als Kontrolle.

Die Wirkung der Behandlung wird mit einer anerkannten Standard-Technik bestimmt. Dabei wird das Vliess 5 mal auf jeder Seite von Hand ca 25 cm in der Länge geteilt. Die Stellen werden so gewählt, dass eine Beobachtung in der Mitte und je zwei weitere Beobachtungen weiter vorne, bzw. weiter hinten möglich sind. Die Gesamtzahl der an diesen Stellen gezählten Läuse wird als Mass für die Lausinfestation dieses Schafs genommen. Diese Methode trägt der Tatsache Rechnung, dass Läuse oft unregelmässig über das Tier verteilt sind. Werden keine Läuse gefunden, wird das ganze Vliess nach Läusen abgesucht.

Unter den vorgenannten Bedingungen erzielten 2 ml der Formulierung F1/ 10 kg Körpergewicht eine Beseitigung (Eradikation) der Schaflausinfestation im Zeitraum von 3 Wochen.

Der Schutz gegen Reinfestation, der durch die gemeinsame Haltung der behandelten mit stark infizierten Tieren bestimmt wird, hielt 17 Wochen an.

Beispiel B2: Anwendung gegen Schaflaus und Schaflausfliege

In einem Experiment in Grossbritannien werden 6 weibliche Schafe der Rasse Welsh Hill im Alter von 1-2 Jahren, die eine natürliche Infestation von Damalinia ovis und Melophagus ovinus (Schaflausfliege) aufwiesen, verwendet.

Applikationsart und Bestimmungstechnik sind die gleichen wie unter B1) beschrieben. Zwei Wochen nach Behandlung mit 2 ml der Formulierung F1/10 kg Körpergewicht werden keine Läuse mehr gefunden, während nach 3 Wochen keine Schaflausfliegen mehr feststellbar sind.

Der Schutz gegen Reinfestation mit Läusen dauerte 14 Wochen.

Beispiel B3: Verwendung gegen Schaflaus und Schaflausfliege

In einem parallelen Experiment werden unter den gleichen Bedingungen wie unter B2) beschrieben die Schafe mit 5 ml der Formulierung F3/10 kg Körpergewicht behandelt.
Die biologische Wirksamkeit beträgt wie unter B2) beschrieben 2 Wochen Eradikation der Läuse, 3 Wochen Eradikation der Schaflausfliege und 14 Wochen Schutz vor Reinfestation gegen Läuse.

Beispiel B4: Verwendung gegen Schaflaus

In einem Experiment in Australien wird eine Gruppe von 20 Schafen, teils Merino, teils Kreuzungen von Merino mit anderen Rassen von gemischtem Alter, nach dem Scheren gegen Damalinia ovis mit 2 ml der Formulierung F4/10 kg Körpergewicht durch Aufguss entlang der Rückenlinie behandelt. Als Bestimmungstechnik wird die gleiche wie unter B1) verwendet.

Die Merino-Kreuzungen sind 1 Woche nach der Behandlung lausfrei. Bei Merinoschafen wird eine 99,9 %-ige Reduktion der Läuse in 4 Wochen nach Behandlung festgestellt; 8 Wochen nach Behandlung sind die Merinos lausfrei. Im Test für Reinfektion können nach 14 Wochen noch immer keine Läuse festgestellt werden.

Beispiel B5: Verwendung gegen Schweineräude

In einem weiteren Experiment in Brasilien wird die Wirkung gegen Sarcoptes suis (Schweineräude) bestimmt.
Eine Gruppe von natürlich infizierten Schweinen von 30-50 kg Körpergewicht wird zweimal im Abstand von 10 Tagen mit je 5 ml der Formulierung F3/10 kg Körpergewicht durch Aufguss entlang der Rückenlinie behandelt. 5 Wochen nach der letzten Behandlung können keine lebenden Milben mehr nachgewiesen werden.

**Ansprüche**
**Patentansprüche für folgende Vertragsstaaten: AT, BE, CH, DE, FR, GB, IT, LI, LU, NL, SE**

1. Nicht-wässrige Pour-on-Flüssigformulierung von ektoparasitiziden Wirkstoffen zur Verwendung am Nutztier, dadurch gekennzeichnet, dass sie neben dem Wirkstoff oder Wirkstoffgemisch als Hauptkomponente Diacetonalkohol und gegebenenfalls als Nebenkomponente einen oder mehrere zweckdienliche, andere Formulierungshilfsstoffe enthält.

2. Nicht-wässrige Pour-on-Formulierung nach Anspruch 1, dadurch gekennzeichnet, dass der Gehalt an Diacetonalkohol ein Vielfaches des Gehaltes an anderen Formulierungshilfsstoffen beträgt.

3. Nicht-wässrige Pour-on-Formulierung nach Anspruch 2, dadurch gekennzeichnet, dass sie 0,1 bis 60 Gewichtsprozent des Wirkstoffs, 40 bis 99,9 Gewichtsprozent Diacetonalkohol und 0 bis 20 Gewichtsprozent an anderen Formulierungshilfsstoffen enthält.

4. Nicht-wässrige Pour-on-Formulierung nach Anspruch 3, dadurch gekennzeichnet, dass man als andere Formulierungshilfsstoffe Cosolventien, Stabilisatoren, Farbstoffe, Antioxidantien, oberflächenaktive Verbindungen oder Haftvermittler einsetzt.

5. Nicht-wässrige Pour-on-Formulierung nach einem der Ansprüche 1 bis 4, dadurch gekennzeichnet, dass man einen ektoparasitiziden Wirkstoff aus der Gruppe der Organophosphorverbindungen, Organochlorverbindungen, Pyrethroide, Carbamate, Amidine, Dimiloide, Cyanopyrimidine oder Triazine einsetzt.

6. Nicht-wässrige Pour-on-Flüssigformulierung nach Anspruch 5, dadurch gekennzeichnet, dass sie als ektoparasitiziden Wirkstoff eine Substanz ausgewählt aus der Reihe
AMITRAZ = N,N-bis-(2,4-Xylyliminomethyl)methylamin;
PROPETAMPHOS = 1-Methylethyl-(E)-3-[[(ethyl-amino)methoxyphosphino-thioyl]-oxy]-2-butensäureester;
CHLORPYRIFOS = Thiophosphorsäure-O,O-diethyl-O-(3,5,6-trichloro-2-pyridinyl)ester;
DIAZINON = Thiophosphorsäure-O,O-diethyl-O-[6-methyl-2-(1-methylethyl)-4-pyrimidinyl]ester;

COUMAPHOS = Thiophosphorsäure-O-(3-chlor-4-methyl-2-oxo-2H-1-benzopyran-7-yl)-O,O-diethylester;
MALATHION = [(Dimethoxyphosphinothioyl)thio]-bernsteinsäurediethylester;
CHLORFENVINPHOS = Phosphorsäure-2-chlor-1-(2,4-dichlorphenyl)ethenyl-diethylester;
LINDANE = 1α, 2α, 3β, 4α, 5α, 6β-Hexachlorcyclohexan;
TIFATOL = N(2,3-Dihydro-3-methyl-1,3-thiazol-2-yliden)-2,4-xylidin;
PHOXIM = O-(α-Cyanobenzylidenamino)-O'-O"-diethylthiophosphat;
FLUMETHRIN = 3-[2-(4-Chlorphenyl)-2-chlorvinyl]-2,2-dimethylcyclopropancarbonsäure-(α-cyano-4-fluor-3-phenoxy-benzylester;
CYROMAZIN = 2-Cyclopropylamino-4, 6-diamino-s-triazin;
CYHALOTHRIN = (R,S)-α-Cyano-3-phenoxybenzyl(Z)-(1RS,3RS)-(2-chlor-3,3,3-trifluorpropenyl)-2,2-dimethylcyclopropancarboxylat;
DELTAMETHRIN = (S)-α-Cyano-3-phenoxybenzyl-(1R,3R)-3-(2,2-dibromovinyl)-2,2-dimethylcyclopropancarboxylat;
FENVALERAT = (RS)-α-Cyano-3-phenoxybenzyl-(RS)-2-(4-chlorphenyl)-3-methylbutyrat;
PERMETHRIN = 3-Phenoxybenzyl-(1RS)-cis,trans-3-(2,2-dichlorvinyl)-2,2-di-methylcyclopropancarboxylat;
CYPERMETHRIN = (RS)-α-cyano-3-phenoxybenzyl(1RS)-cis,trans-3-(2,2-dichlorvinyl) -2, 2-dimethylcyclopropancarboxylat;
FAMPHUR = O-[4-(Dimethylsulfamoyl)-phenyl]-O,O-dimethylphosphorthioat;
FENTHION = O,O-Dimethyl-O-4-methylthio-m-tolylphosphorthioat;
PHOSMET = O,O-Dimethyl-S-phthalimidomethylphosphorodithioat;
TETRACHLORVINPHOS = [(Z)-2-Chlor-1-(2,4,5-trichlorphenyl)-vinyl]-dimethylphosphat;
PROMACYL = 5-Methyl-m-cumenylbutyryl(methyl)carbamat; und
DIFLUBENZURON = 1-(4-Chlorphenyl)-3-(2,6-difluorbenzoyl)-harnstoff enthält.

7. Nicht-wässrige Pour-on-Formulierung nach Anspruch 6, dadurch gekennzeichnet, dass man als Wirkstoff Diazinon einsetzt.

8. Nicht-wässrige Pour-on-Formulierung nach Anspruch 6, dadurch gekennzeichnet, dass man als Wirkstoff Cypermethrin einsetzt.

9. Nicht-wässrige Pour-on-Flüssigformulierung nach Anspruch 5, dadurch gekennzeichnet, dass sie als ektoparasitiziden Wirkstoff eine Substanz der Formel X enthält,

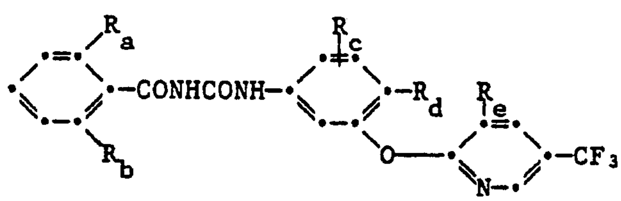

(X)

worin

- $R_a$ für Wasserstoff, $C_1$-$C_2$-Alkoxy, $C_1$-$C_2$-Alkylthio, $C_1$-$C_2$-Alkyl oder Halogen steht;
- $R_b$ Wasserstoff oder Halogen bedeutet;
- $R_c$ für Wasserstoff oder Halogen steht;
- $R_d$ Wasserstoff, $C_1$-$C_2$-Alkyl oder Halogen bedeutet; und
- $R_e$ für Halogen steht.

10. Formulierung nach Anspruch 9, dadurch gekennzeichnet, dass sie als Wirkstoff eine Substanz enthält, ausgewählt aus der Reihe:

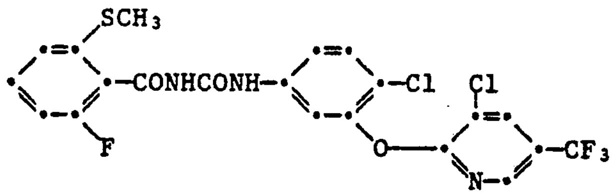

,

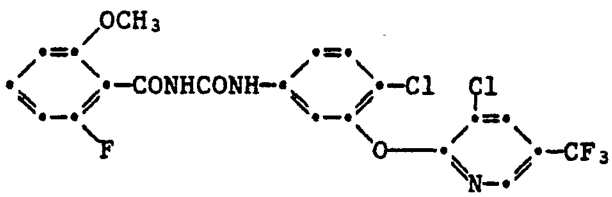

,

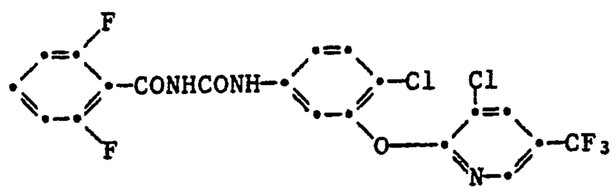

,

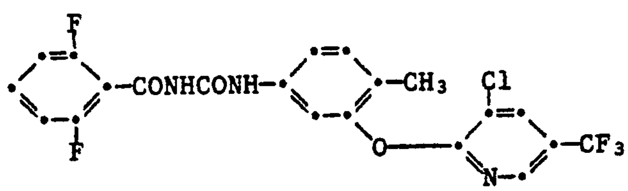

,

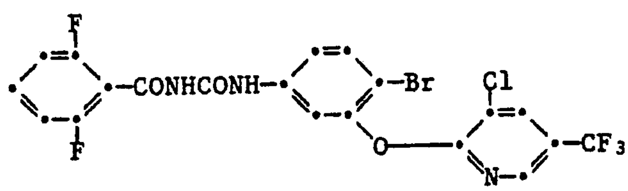

,

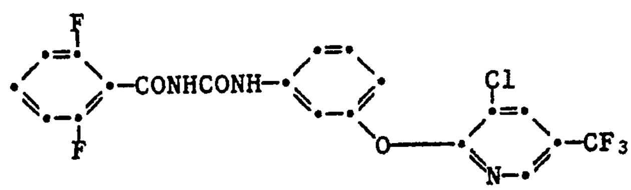

und

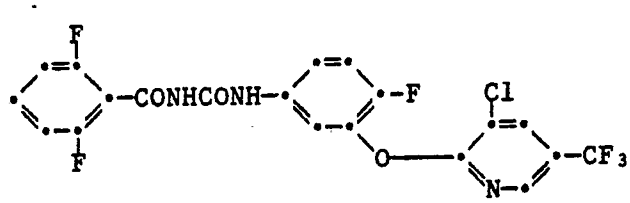

.

11. Nicht-wässrige Pour-on-Flüssigformulierung nach Anspruch 5, dadurch gekennzeichnet, dass sie als ektoparasitiziden Wirkstoff eine Substanz der Formel XX enthält

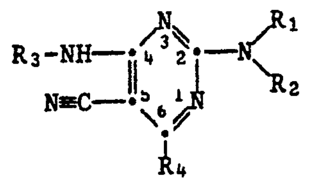

(XX),

worin

$R_1$ Wasserstoff, $C_1$-$C_6$-Alkyl, $C_2$-$C_6$-Alkenyl oder $C_2$-$C_6$-Alkinyl;

$R_2$ Wasserstoff, $C_1$-$C_{10}$-Alkyl oder $C_3$-$C_6$-Cycloalkyl; oder

$R_1$ und $R_2$ zusammen einen der Reste $-(CH_2)_3-$, $-(CH_2)_4-$ oder $-(CH_2)_5-$;

$R_3$ Wasserstoff oder einen der Reste

$-CO-R_5$ oder $-SO_2-R_6$ ;

$R_4$ einen der Reste

$-NH_2$, $-NH-CO-R_5$, $-NH-SO_2-R_6$ ,

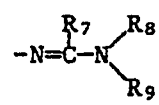

oder $-NH-CH=N-R_{10}$ ;

$R_5$ Wasserstoff, $C_1$-$C_{12}$-Alkyl, $C_3$-$C_6$-Cycloalkyl, $C_1$-$C_6$-Alkoxy, perhalogeniertes $C_1$-$C_3$-Alkyl oder den Rest

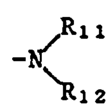

;

$R_6$ $C_1$-$C_6$-Alkyl;

$R_7$ Wasserstoff oder $C_1$-$C_6$-Alkyl;

$R_8$ und $R_9$ unabhängig voneinander Wasserstoff oder $C_1$-$C_6$-Alkyl oder zusammen einen der Reste $-(CH_2)_3-$, $-(CH_2)_4-$ oder $-(CH_2)_5-$;

$R_{10}$ einer der Reste $-SO_2-R_{13}$ oder

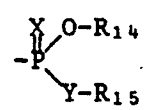

;

$R_{11}$ und $R_{12}$ unabhängig voneinander Wasserstoff oder $C_1$-$C_6$-Alkyl oder zusammen einen der Reste $-(CH_2)_3-$, $-(CH_2)_4-$ oder $-(CH_2)_5-$;

$R_{13}$ $C_1$-$C_{10}$-Alkyl, mit bis zu 10 Halogenatomen substituiertes $C_1$-$C_{10}$-Alkyl oder $C_3$-$C_6$-Cycloalkyl;

$R_{14}$ und $R_{15}$ unabhängig voneinander $C_1$-$C_{10}$-Alkyl;

X und Y unabhängig voneinander Sauerstoff oder Schwefel bedeuten, oder ein Salz davon.

**12.** Verfahren zur Herstellung einer nicht-wässrigen Pour-on-Flüssigformulierung nach Anspruch 1, dadurch gekennzeichnet, dass man den ektoparasitiziden Wirkstoff in Diacetonalkohol löst oder gegebenenfalls

unter Zusatz von oberflächenaktiven Verbindungen emulgiert oder suspendiert und, falls erforderlich, die anderen Hilfsingredienzien vorlegt oder der Mischung zusetzt.

13. Verwendung von Diacetonalkohol zur Herstellung von nicht-wässrigen Pour-on-Flüssigformulierungen.

14. Nicht-wässrige Pour-on-Formulierung gemäss Anspruch 1 zur Bekämpfung von Ektoparasiten an Nutztieren.

**Patentansprüche für folgende Vertragsstaaten: ES, GR**

1. Verfahren zur Herstellung einer nicht-wässrigen Pour-on-Flüssigformulierung von ektoparasitiziden Wirkstoffen zur Verwendung am Nutztier, die neben dem Wirkstoff oder Wirkstoffgemisch als Hauptkomponente Diacetonalkohol und gegebenenfalls als Nebenkomponente einen oder mehrere zweckdienliche, andere Formulierungshilfsstoffe enthält, dadurch gekennzeichnet, dass man den ektoparasitiziden Wirkstoff in Diacetonalkohol löst oder gegebenenfalls unter Zusatz von oberflächenaktiven Verbindungen emulgiert oder suspendiert und, falls erforderlich, die übrigen Hilfsingredienzien vorlegt oder der Mischung zusetzt.

2. Verfahren zur Herstellung einer nicht-wässrigen Pour-on-Formulierung nach Anspruch 1, dadurch gekennzeichnet, dass der Gehalt an Diacetonalkohol ein Vielfaches des Gehaltes an anderen Formulierungshilfsstoffen beträgt.

3. Verfahren zur Herstellung einer nicht-wässrigen Pour-on-Formulierung nach Anspruch 2, dadurch gekennzeichnet, dass sie 0,1 bis 60 Gewichtsprozent des Wirkstoffs, 40 bis 99,9 Gewichtsprozent Diacetonalkohol und 0 bis 20 Gewichtsprozent an anderen Formulierungshilfsstoffen enthält.

4. Verfahren zur Herstellung einer nicht-wässrigen Pour-on-Formulierung nach Anspruch 3, dadurch gekennzeichnet, dass man als andere Formulierungshilfsstoffe Cosolventien, Stabilisatoren, Farbstoffe, Antioxidantien, oberflächenaktive Verbindungen oder Haftvermittler einsetzt.

5. Verfahren zur Herstellung einer nicht-wässrigen Pour-on-Formulierung nach einem der Ansprüche 1 bis 4, dadurch gekennzeichnet, dass man einen ektoparasitiziden Wirkstoff aus der Gruppe der Organophosphorverbindungen, Organochlorverbindungen, Pyrethroide, Carbamate, Amidine, Dimiloide, Cyanopyrimidine oder Triazine einsetzt.

6. Verfahren zur Herstellung einer nicht-wässrigen Pour-on-Flüssigformulierung nach Anspruch 5, dadurch gekennzeichnet, dass sie als ektoparasitiziden Wirkstoff eine Substanz ausgewählt aus der Reihe
AMITRAZ = N,N-bis(2,4-Xylyliminomethyl)methylamin;
PROPETAMPHOS = 1-Methylethyl-(E)-3-[(ethyl-amino)methoxyphosphinothioyl]-oxy]-2-butensäureester;
CHLORPYRIFOS = Thiophosphorsäure-O,O-diethyl-O-(3,5,6-trichloro-2-pyridinyl)ester;
DIAZINON = Thiophosphorsäure-O,O-diethyl-O-[6-methyl-2-(1-methylethyl)-4-pyrimidinyl]ester;
COUMAPHOS = Thiophosphorsäure-O-(3-chlor-4-methyl-2-oxo-2H-1-benzopyran-7-yl)-O,O-diethylester;
MALATHION = [(Dimethoxyphosphinothioyl)thio]-bernsteinsäurediethylester;
CHLORFENVINPHOS = Phosphorsäure-2-chlor-1-(2,4-dichlorphenyl)ethenyl-diethylester;
LINDANE = 1α, 2α, 3β, 4α, 5α, 6β-Hexachlorcyclohexan;
TIFATOL = N-(2,3-Dihydro-3-methyl-1,3-thiazol-2-yliden)-2,4-xylidin;
PHOXIM = O(α-Cyanobenzylidenamino)-O'-O"-diethylthiophosphat;
FLUMETHRIN = 3-[2-(4-Chlorphenyl)-2-chlorvinyl]-2,2-dimethylcyclopropancarbonsäure-(α-cyano-4-fluor-3-phenoxy-benzylester;
CYROMAZIN = 2-Cyclopropylamino-4,6-diamino-s-triazin;
CYHALOTHRIN = (R,S)-α-Cyano-3-phenoxybenzyl(Z)-(1RS,3RS)-(2-chlor-3,3,3-trifluorpropenyl)-2,2-dimethylcyclopropancarboxylat;
DELTAMETHRIN = (S)-α-Cyano-3-phenoxybenzyl-(1R,3R)-3-(2,2-dibromovinyl)-2,2-dimethylcyclopropancarboxylat;
FENVALERAT = (RS)-α-Cyano-3-phenoxybenzyl-(RS)-2-(4-chlorphenyl)-3-methylbutyrat;
PERMETHRIN = 3-Phenoxybenzyl-(1RS)cis,trans-3-(2,2-dichlorvinyl)-2,2-dimethylcyclopropancarbo-

xylat;
CYPERMETHRIN = (RS)-α-cyano-3-phenoxybenzyl(1RS)-cis,trans-3-(2,2-dichlorvinyl)-2,2-dimethylcyclopropancarboxylat;
FAMPHUR = O-[4-(Dimethylsulfamoyl)-phenyl]-O,O-dimethylphosphorthioat;
FENTHION = O,O-Dimethyl-O-4-methylthio-m-tolylphosphorthioat;
PHOSMET = O,O-Dimethyl-S-phthalimidomethylphosphorodithioat;
TETRACHLORVINPHOS = [(Z)2-Chlor-1-(2,4,5-trichlorphenyl)-vinyl]-dimethylphosphat;
PROMACYL = 5-Methyl-m-cumenylbutyryl(methyl)carbamat; und
DIFLUBENZURON = 1-(4-Chlorphenyl)-3-(2,6-difluorbenzoyl)-harnstoff enthält.

7. Verfahren zur Herstellung einer nicht-wässrigen Pour-on-Formulierung nach Anspruch 6, dadurch gekennzeichnet, dass man als Wirkstoff Diazinon einsetzt.

8. Verfahren zur Herstellung einer nicht-wässrigen Pour-on-Formulierung nach Anspruch 6, dadurch gekennzeichnet, dass man als Wirkstoff Cypermethrin einsetzt.

9. Verfahren zur Herstellung einer nicht-wässrigen Pour-on-Flüssigformulierung nach Anspruch 5, dadurch gekennzeichnet, dass sie als ektoparasitiziden Wirkstoff eine Substanz der Formel X enthält,

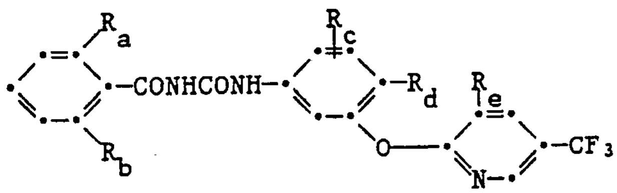

(X)

worin

$R_a$ für Wasserstoff, $C_1$-$C_2$-Alkoxy, $C_1$-$C_2$-Alkylthio, $C_1$-$C_2$-Alkyl oder Halogen steht;
$R_b$ Wasserstoff oder Halogen bedeutet;
$R_c$ für Wasserstoff oder Halogen steht;
$R_d$ Wasserstoff, $C_1$-$C_2$-Alkyl oder Halogen bedeutet; und
$R_e$ für Halogen steht.

10. Verfahren zur Herstellung einer nicht-wässrigen Pour-on-Flüssigformulierung nach Anspruch 9, dadurch gekennzeichnet, dass sie als Wirkstoff eine Substanz enthält, ausgewählt aus der Reihe:

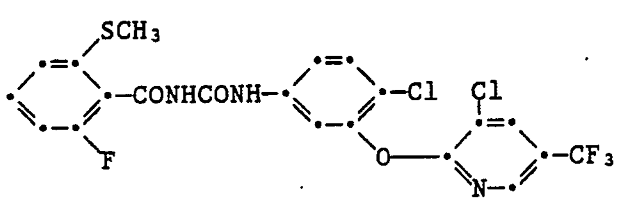

,

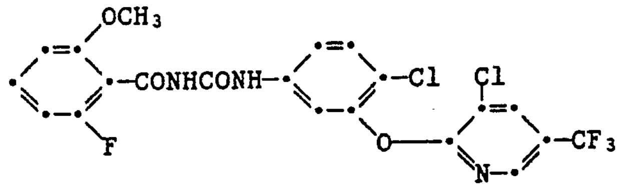 ,

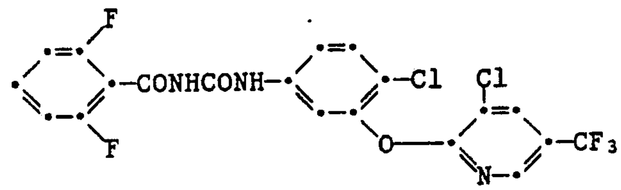 ,

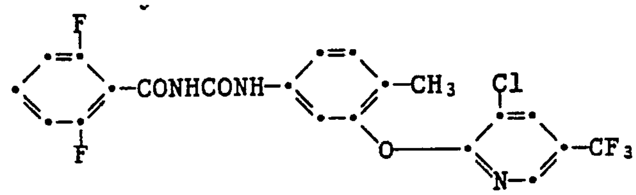 ,

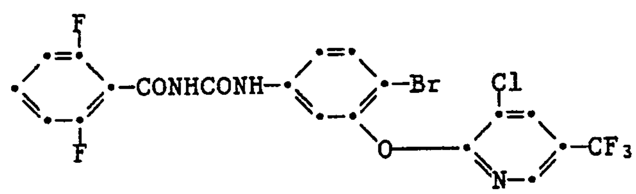 ,

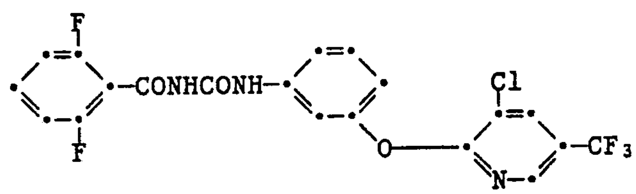

und

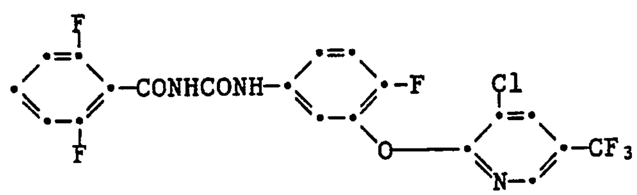 .

11. Verfahren zur Herstellung einer nicht-wässrigen Pour-on-Flüssigformulierung nach Anspruch 5, dadurch gekennzeichnet, dass sie als ektoparasitiziden Wirkstoff eine Substanz der Formel XX enthält

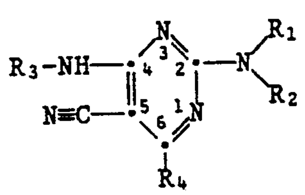 (XX),

worin

$R_1$ Wasserstoff, $C_1$-$C_6$-Alkyl, $C_2$-$C_6$-Alkenyl oder $C_2$-$C_6$-Alkinyl;
$R_2$ Wasserstoff, $C_1$-$C_{10}$-Alkyl oder $C_3$-$C_6$-Cycloalkyl; oder
$R_1$ und $R_2$ zusammen einen der Reste $-(CH_2)3-$, $-(CH_2)4-$ oder $-(CH_2)_5-$; $R_3$ Wasserstoff oder einen der Reste

$-CO-R_5$ oder $-SO_2-R_6$ ;

$R_4$ einen der Reste

$-NH_2$, $-NH-CO-R_5$, $-NH-O0_2-R_6$ ,

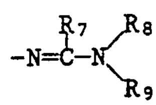

oder $-NH-CH=N-R_{10}$ ;
$R_5$ Wasserstoff, $C_1$-$C_{12}$-Alkyl, $C_3$-$C_6$-Cycloalkyl, $C_1$-$C_6$-Alkoxy, perhalogeniertes $C_1$-$C_3$-Alkyl oder den Rest

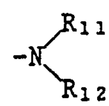 ;

$R_6$ $C_1$-$C_6$-Alkyl;
$R_7$ Wasserstoff oder $C_1$-$C_6$-Alkyl;
$R_8$ und $R_9$ unabhängig voneinander Wasserstoff oder $C_1$-$C_6$-Alkyl oder zusammen einen der Reste $-(cH_2)_3-$, $-(CH_2)_4-$ oder $-(CH_2)_5-$;
$R_{10}$ einer der Reste $-SO_2-R_{13}$ oder

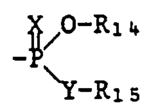 ;

$R_{11}$ und $R_{12}$ unabhängig voneinander Wasserstoff oder $C_1$-$C_6$-Alkyl oder zusammen einen der Reste $-(CH_2)_3-$, $-(CH_2)_4-$ oder $-(CH_2)_5-$;
$R_{13}$ $C_1$-$C_{10}$-Alkyl, mit bis zu 10 Halogenatomen substituiertes $C_1$-$C_{10}$-Alkyl oder $C_3$-$C_6$-Cycloalkyl;
$R_{14}$ und $R_{15}$ unabhängig voneinander $C_1$-$C_{10}$-Alkyl;
X und Y unabhängig voneinander Sauerstoff oder Schwefel bedeuten, oder
ein Salz davon.

**12.** Verwendung von Diacetonalkohol zur Herstellung von nicht-wässrigen Pour-on-Flüssigformulierungen.

## Claims

**Claims for the following Contracting States: AT, BE, CH, DE, FR, GB, IT, LI, LU, NL, SE.**

**1.** A non-aqueous pour-on liquid formulation of ectoparasiticidal active ingredients for application to productive livestock, which formulation comprises, in addition to the active ingredient or mixture of active ingredients, diacetone alcohol as main component and if necessary, one or more other useful formulation assistants as subsidiary component.

**2.** A non-aqueous pour-on formulation according to claim 1, wherein the content of diacetone alcohol is a

multiple of the content of the other formulation assistants.

3. A non-aqueous pour-on formulation according to claim 2, which comprises 0.1 to 60 per cent by weight of the active ingredient, 40 to 99.9 per cent by weight of diacetone alcohol, and 0 to 20 per cent by weight of other formulation assistants.

4. A non-aqueous pour-on formulation according to claim 3, wherein the other formulation assistants employed are cosolvents, stabilisers, dyes, antioxidants, surface-active compounds or adhesion promoters.

5. A non-aqueous pour-on formulation according to any one of claims 1 to 4, wherein an ectoparasiticidal active ingredient selected from the group consisting of organophosphorus compounds, organochlorine compounds, pyrethroids, carbamates, amidines, dimiloids, cyanopyrimidines or triazines is employed.

6. A non-aqueous pour-on liquid formulation according to claim 5, wherein the ectoparasiticidal active ingredient is a substance selected from the group consisting of
AMITRAZ = N,N-bis(2,4-xylyliminomethyl)methylamine;
PROPETAMPHOS = 1-methylethyl-(E)-3-[[(ethylamino)methoxyphosphinothioyl]oxy]-2-butenoic acid ester;
CHLORPYRIFOS = 0,0-diethyl-0-(3,5,6-trichloropyridin-2-yl) thiophosphate;
DIAZINON = 0,0-diethyl-0-[6-methyl-2-(1-methylethyl)-4-pyrimidinyl] thiophosphate;
COUMAPHOS = 0-(3-chloro-4-methyl-2-oxo-2H-1-benzopyran-7-yl)-0,0-diethyl thiophosphate;
MALATHION = diethyl[(dimethoxyphosphinothioyl)thio]succinate;
CHLORFENVINPHOS = 2-chloro-1-(2,4-dichlorophenyl)ethenyldiethyl phosphate;
LINDANE = 1α,2α,3β,4α,5α,6β-hexachlorocyclohexane;
TIFATOL = N-(2,3-dihydro-3-methyl-1,3-thiazol-2-ylidine)-2,4-xylidine;
PHOXIM = 0-(α-cyanobenzylideneamino)-0'-0"-diethyl thiophosphate;
FLUMETHRIN = α-cyano-4-fluoro-3-phenoxybenzyl 3-[2-(4-chlorophenyl)-2-chlorovinyl]- 2,2-dimethylcyclopropanecarboxylate;
CYROMAZIN = 2-cyclopropylamino-4,6-diamino-s-triazine;
CYHALOTHRIN = (R,S)-α-cyano-3-phenoxybenzyl (Z)-(1RS,3RS)-(2-chloro-3,3,3 - trifluoropropenyl)-2,2-dimethylcyclopropanecarboxylate;
DELTAMETHRIN = (S)-α-cyano-3-phenoxybenzyl (1R,3R)-3-(2,2-dibromovinyl)-2,2-dimethylcyclopropanecarboxylate;
FENVALERAT = (RS)-α-cyano-3-phenoxybenzyl (RS)-2-(4-chlorophenyl)-3 -methylbutyrate;
PERMETHRIN = 3-phenoxybenzyl (1RS)-cis,trans-3-(2,2-dichlorovinyl)-2,2 - dimethylcyclopropanecarboxylate;
CYPERMETHRIN = (RS)-α-cyano-3-phenoxybenzyl (1RS)-cis,trans-3-(2,2-dichlorovinyl)-2,2-dimethylcyclopropanecarboxylate;
FAMPHUR = 0- [4- (dimethylsulfamoyl)phenyl] 0,0-dimethylphosphorothioate;
FENTHION = 0,0-dimethyl 0-4-methylthio-m-tolylphosphorothioate;
PHOSMET = 0,0-dimethyl S-phthalimidomethyl phosphorodithioate;
TETRACHLORVINPHOS = [(Z)-2-chloro-1-(2,4,5-trichlorophenyl)vinyl]-dimethylphosphate;
PROMACYL = 5 -methyl-m-cumenyl butyryl(methyl) carbamate; and
DIFLUBENZRON = 1-(4-chlorophenyl)-3-(2,6-difluorobenzoyl)urea.

7. A non-aqueous pour-on formulation according to claim 6, wherein diazinon is employed as active ingredient.

8. A non-aqueous pour-on formulation according to claim 6, wherein cypermethrin is employed as active ingredient.

9. A non-aqueous pour-on liquid formulation according to claim 5, wherein the ectoparasiticidal active ingredient is a substance of the formula X

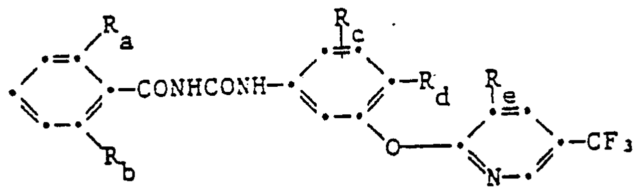 (X)

in which

- $R_a$ is hydrogen, $C_1$-$C_2$alkoxy, $C_1$-$C_2$alkylthio, $C_1$-$C_2$alkyl or halogen;
- $R_b$ is hydrogen or halogen;
- $R_c$ is hydrogen or halogen;
- $R_d$ is hydrogen, $C_1$-$C_2$alkyl or halogen; and
- $R_e$ is halogen.

**10.** A formulation according to claim 9, wherein the active ingredient is a substance selected from the group consisting of

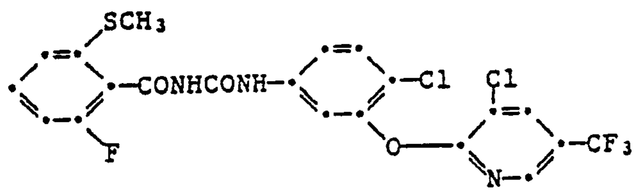 ,

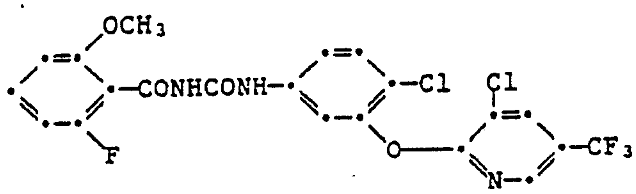 ,

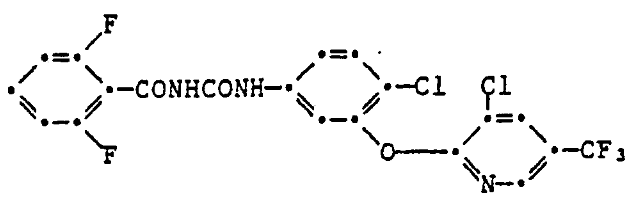 ,

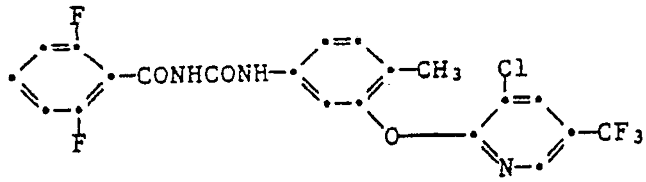

,

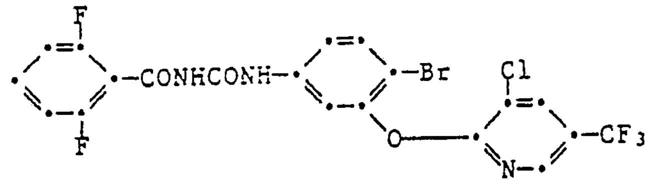

,

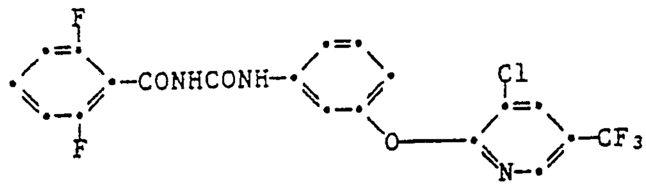

and

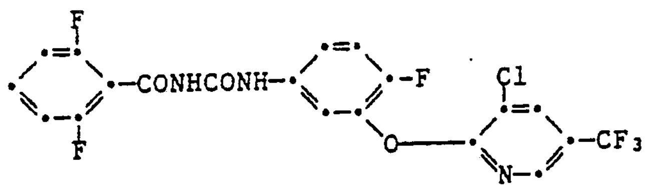

.

11. A non-aqueous pour-on liquid formulation according to claim 5, wherein the ectoparasiticidal active ingredient is a substance of the formula XX

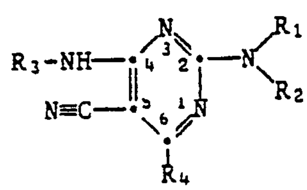

(XX)

in which

| | |
|---|---|
| $R_1$ | is hydrogen, $C_1$-$C_6$alkyl, $C_2$-$C_6$alkenyl or $C_2$-$C_6$alkynyl; |
| $R_2$ | is hydrogen, $C_1$-$C_{10}$alkyl or $C_3$-$C_6$cycloalkyl; or |
| $R_1$ and $R_2$ | together are a radical selected from the group consisting of $-(CH_2)_3-$, $-(CH_2)_4-$ or $-(CH_2)_5-$; |
| $R_3$ | is hydrogen or a radical $-CO-R_5$ or $-SO_2-R_6$; |
| $R_4$ | is a radical selected from the group consisting of |

$-NH_2$, $-NH-CO-R_5$, $-NH-SO_2-R_6$,

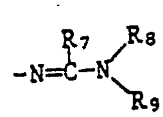

or $-NH-CH-N-R_{10}$;

$R_5$ is hydrogen, $C_1$-$C_{12}$alkyl, $C_3$-$C_6$cycloalkyl, $C_1$-$C_6$alkoxy, perhalogenated $C_1$-$C_3$alkyl or the radical

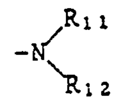

$R_6$ is $C_1$-$C_6$alkyl;

$R_7$ is hydrogen or $C_1$-$C_6$alkyl;

$R_8$ and $R_9$ are each independently of the other hydrogen or $C_1$-$C_6$alkyl or together are a radical selected from the group consisting of $-(CH_2)_3-$, $-(CH_2)_4-$ or $-(CH_2)_5-$;

$R_{10}$ is a radical $-SO_2-R_{13}$ or

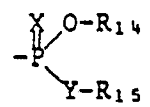

;

$R_{11}$ and $R_{12}$ are each independently of the other hydrogen or $C_1$-$C_6$alkyl or together are a radical selected from the group consisting of $-(CH_2)_3-$, $-(CH_2)_4-$ or $-(CH_2)_5-$;

$R_{13}$ is $C_1$-$C_{10}$alkyl, $C_1$-$C_{10}$alkyl substituted by up to 10 halogen atoms, or is $C_3$-$C_6$cycloalkyl;

$R_{14}$ and $R_{15}$ are each independently of the other $C_1$-$C_{10}$alkyl; and X and Y are each independently of the other oxygen or sulfur, or a salt thereof.

**12.** A process for the preparation of a non-aqueous pour-on liquid formulation according to claim 1, which comprises dissolving or, if appropriate with the addition of a surface-active compound, emulsifying or suspending the ectoparasiticidal active ingredient in diacetone alcohol and, if necessary, initially introducing the other assistant ingredients or adding them to the mixture.

**13.** Use of diacetone alcohol for the preparation of a non-aqueous pour-on liquid formulation.

**14.** A non-aqueous pour-on formulation according to claim 1 for controlling ectoparasites on productive livestock.

**Claims for the following Contracting States: ES,GR.**

**1.** A process for the preparation of a non-aqueous pour-on liquid formulation of ectoparasiticidal active ingredients for application to productive livestock, which formulation comprises, in addition to the active ingredient or mixture of active ingredients, diacetone alcohol as main component and if necessary, one or more other useful formulation assistants as subsidiary component, which comprises dissolving or, with the appropriate addition of a surface-active compound, emulsifying or suspending, the ectoparasiticidal active ingredient in diacetone alcohol and, if necessary, initially inroducing the other assistant ingredients or adding them to the mixture.

**2.** A process for the preparation of a non-aqueous pour-on formulation according to claim 1, wherein the content of diacetone alcohol is a multiple of the content of the other formulation assistants.

**3.** A process for the preparation of a non-aqueous pour-on formulation according to claim 2, which comprises 0.1 to 60 per cent by weight of the active ingredient, 40 to 99.9 per cent by weight of diacetone alcohol, and 0 to 20 per cent by weight of other formulation assistants.

**4.** A process for the preparation of a non-aqueous pour-on formulation according to claim 3, wherein the other formulation assistants employed are cosolvents, stabilisers, dyes, antioxidants, surface-active

compounds or adhesion promoters.

5. A process for the preparation of a non-aqueous pour-on formulation according to any one of claims 1 to 4, wherein an ectoparasiticidal active ingredient selected from the group consisting of organophosphorus compounds, organochlorine compounds, pyrethroids, carbamates, amidines, dimiloids, cyanopyrimidines or triazines is employed.

6. A process for the preparation of a non-aqueous pour-on liquid formulation according to claim 5, wherein the ectoparasiticidal active ingredient is substance selected from the group consisting of
AMITRAZ = N,N-bis(2,4-xylyliminomethyl)methylamine;
PROPETAMPHOS = 1-methylethyl-(E)-3-[[(ethylamino)methoxyphosphinothioyl]oxy]-2-butenoic acid ester;
CHLORPYRIFOS = 0,0-diethyl-0-(3,5,6-trichloropyridin-2-yl) thiophosphate;
DIAZINON = 0,0-diethyl-0-[6-methyl-2-(1-methylethyl)-4-pyrimidinyl] thiophosphate;
COUMAPHOS = 0-(3-chloro-4-methyl-2-oxo-2H-1-benzopyran-7-y1)-0,0-diethyl thiophosphate;
MALATHION = diethyl[(dimethoxyphosphinothioyl)thio]succinate;
CHLORFENVINPHOS = 2-chloro-1-(2,4-dichlorophenyl)ethenyldiethyl phosphate;
LINDANE = 1α,2α,3β,4α,5α,6β-hexachlorocyclohexane;
TIFATOL = N-(2,3-dihydro-3-methyl-1,3-thiazol-2-ylidine)-2,4-xylidine;
PHOXIM = 0-(α-cyanobenzylideneamino)-0'-0"-diethyl thiophosphate;
FLUMETHRIN = α-cyano-4-fluoro-3-phenoxybenzyl 3-[2-(4-chlorophenyl)-2-chlorovinyl ] - 2,2-dimethylcyclopropanecarboxylate;
CYROMAZIN = 2-cyclopropylamino-4,6-diamino-s-triazine;
CYHALOTHRIN = (R,S)-α-cyano-3-phenoxybenzyl (Z)-(1RS,3RS)-(2-chloro-3,3,3 - trifluoropropenyl) - 2,2 -dimethylcyclopropanecarboxylate;
DELTAMETHRIN = (S)-α-cyano-3-phenoxybenzyl (1R,3R)-3-(2,2-dibromovinyl)-2,2-dimethylcyclopropanecarboxylate;
FENVALERAT = (RS)-α-cyano-3-phenoxybenzyl (RS)-2-(4-chlorophenyl)-3-methylbutyrate;
PERMETHRIN = 3-phenoxybenzyl (1RS)-cis,trans-3-(2,2-dichlorovinyl)-2,2-dimethylcyclopropanecarboxylate;
CYPERMETHRIN = (RS)-α-cyano-3-phenoxybenzyl (1RS)-cis,trans-3-2,2-dichlorovinyl) - 2,2-dimethylcyclopropanecarboxylate;
FAMPHUR = 0-[4-(dimethylsulfamoyl)phenyl] 0,0-dimethylphosphorothioate;
FENTHION = 0,0-dimethyl 0-4-methylthio-m-tolylphosphorothioate;
PHOSMET = 0,0-dimethyl S-phthalimidomethyl phosphorodithioate;
TETRACHLORVINPHOS = [(Z)-2-chloro-1-(2,4,5-trichlorophenyl)vinyl]-dimethylphosphate;
PROMACYL = 5-methyl-m-cumenyl butyryl(methyl)carbamate; and
DIFLUBENZURON = 1-(4-chlorophenyl)-3-(2,6-difluorobenzoyl)urea.

7. A process for the preparation of a non-aqueous pour-on formulation according to claim 6, wherein diazinon is employed as active ingredient.

8. A process for the preparation of a non-aqueous pour-on formulation according to claim 6, wherein cypermethrin is employed as active ingredient.

9. A process for the preparation of a non-aqueous pour-on liquid formulation according to claim 5, wherein the ectoparasiticidal active ingredient is a substance of the formula X

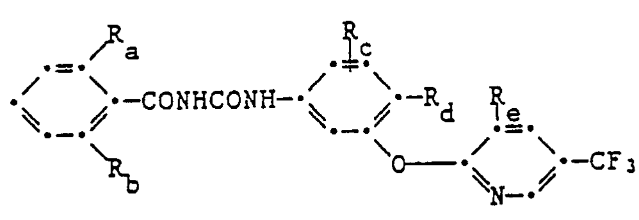

(X)

in which

| | |
|---|---|
| $R_a$ | is hydrogen, $C_1$-$C_2$alkoxy, $C_1$-$C_2$alkylthio, $C_1$-$C_2$alkyl or halogen; |
| $R_b$ | is hydrogen or halogen; |
| $R_c$ | is hydrogen or halogen; |
| $R_d$ | is hydrogen, $C_1$-$C_2$alkyl or halogen; and |
| $R_e$ | is halogen. |

**10.** A process for the preparation of a non-aqueous pour-on liquid formulation according to claim 9, wherein the active ingredient is substance selected from the group consisting of

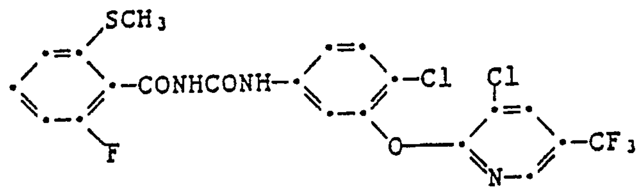

,

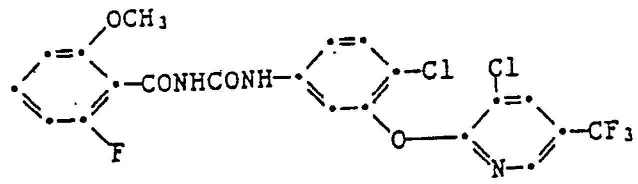

,

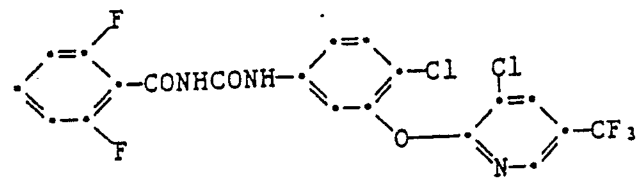

,

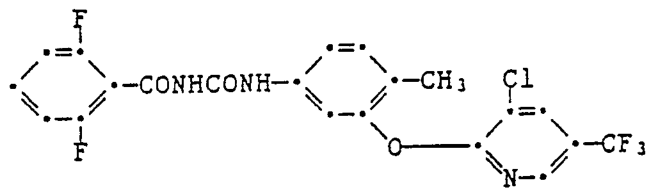

,

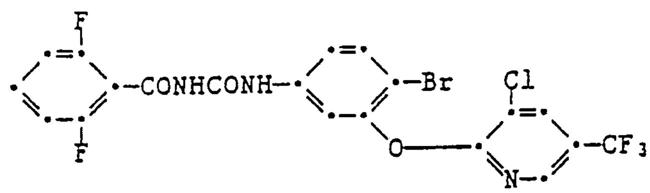

,

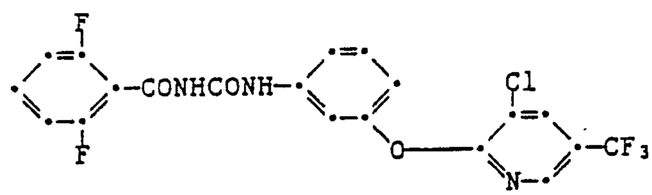

and

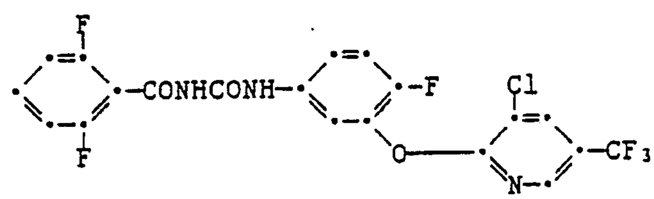

.

11. A process for the preparation of a non-aqueous pour-on liquid formulation according to claim 5, wherein the ectoparasiticidal active ingredient is a substance of the formula XX

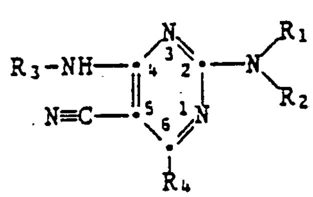

(XX)

in which

$R_1$ is hydrogen, $C_1$-$C_6$alkyl, $C_2$-$C_6$alkenyl or $C_2$-$C_6$alkynyl;
$R_2$ is hydrogen, $C_1$-$C_{10}$alkyl or $C_3$-$C_6$cycloalkyl; or
$R_1$ and $R_2$ together are a radical selected from the group consisting of $-(CH_2)_3-$, $-(CH_2)_4-$ or $-(CH_2)_5-$;
$R_3$ is hydrogen or a radical $-CO-R_5$ or $-SO_2-R_6$;
$R_4$ is a radical selected from the group consisting of $-NH_2$, $-NH-CO-R_5$, $-NH-SO_2-R_6$,

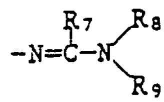

or $-NH-CH-N-R_{10}$;

$R_5$ is hydrogen, $C_1$-$C_{12}$alkyl, $C_3$-$C_6$cycloalkyl, $C_1$-$C_6$alkoxy, perhalogenated $C_1$-$C_3$alkyl or the radical

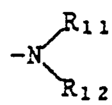 ;

$R_6$ is $C_1$-$C_6$alkyl;
$R_7$ is hydrogen or $C_1$-$C_6$alkyl;
$R_8$ and $R_9$ are each independently of the other hydrogen or $C_1$-$C_6$alkyl or together are a radical selected from the group consisting of $-(CH_2)_3-$, $-(CH_2)_4-$ or $-(CH_2)_5-$;
$R_{10}$ is a radical $-SO_2R_{13}$ or

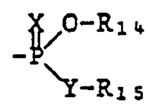 ;

$R_{11}$ and $R_{12}$ are each independently of the other hydrogen or $C_1$-$C_6$alkyl or together are a radical selected from the group consisting of $-(CH_2)_3-$, $-(CH_2)_4-$ or $-(CH_2)_5-$;
$R_{13}$ is $C_1$-$C_{10}$alkyl, $C_1$-$C_{10}$alkyl substituted by up to 10 halogen atoms, or is $C_3$-$C_6$cycloalkyl;
$R_{14}$ and $R_{15}$ are each independently of the other $C_1$-$C_{10}$alkyl; and X and Y are each independently of the other oxygen or sulfur, or a salt thereof.

**12.** Use of diacetone alcohol for the preparation of a non-aqueous pour-on liquid formulation.

**Revendications**
**Revendications pour les Etats contractants suivants : AT, BE, CH, DE, FR, GB, IT, LI, LU, NL, SE**

**1.** Formulation "pour-on" liquide, non aqueuse d'agents actifs ectoparasiticides destinée aux animaux utiles, caractérisée en ce que, outre l'agent actif ou le mélange d'agents actifs, elle contient en tant que constituant principal de l'alcool diacétonique et éventuellement en tant que constituant secondaire un ou plusieurs autres adjuvants de formulation convenables.

**2.** Formulation "pour-on" non aqueuse selon la revendication 1, caractérisée en ce que la teneur en alcool diacétonique est égale à plusieurs fois la teneur en d'autres adjuvants de formulation.

**3.** Formulation "pour-on" non aqueuse selon la revendication 2, caractérisée en ce qu'elle comporte 0,1 à

60% en poids de l'agent actif, 40 à 99,9% en poids d'alcool diacétonique et 0 à 20% en poids d'autres adjuvants de formulation.

4. Formulation "pour-on" non aqueuse selon la revendication 3, caractérisée en ce qu'on introduit comme autres adjuvants de formulation des cosolvants, des agents stabilisants, des colorants, des antioxydants, des agents de surface ou des agents de fixation.

5. Formulation "pour-on" non aqueuse selon l'une quelconque des revendications 1 à 4, caractérisée en ce qu'on utilise un agent actif ectoparasiticide choisi parmi les composés organophosphorés, les composés organochlorés, les pyréthroïdes, les carbamates, les amidines, les dimiloïdes, les cyanopyrimidines ou les triazines.

6. Formulation "pour-on" liquide, non aqueuse selon la revendication 5, caractérisée en ce qu'elle contient en tant qu'agent à activité ectoparasiticide une substance choisie parmi les suivantes :
AMITRAZ = N,N-bis-(2,4-xylyliminométhyl)méthylamine;
PROPETAMPHOS = ester d'acide 1-méthyléthyl-(E)-3-[[(éthyl-amino)méthoxyphosphinothioyl]-oxy] -2-buténique;
CHLORPYRIFOS = thiophosphate de 0,0-diéthyl-0-(3,5,6-trichloro-2-pyridinyle);
DIAZINON = thiophosphate de 0,0-diéthyl-0-[6-méthyl-2-(1-méthyléthyl)-4-pyrimidinyle);
COUHAPHOS = thiophosphate de 0-( 3-chloro-4-méthyl-2-oxo-2H-1-benzopyrann-7-yl) -0,0-diéthyle;
MALATHION = [(diméthoxyphosphinothioyl)thio]-succinate de diéthyle;
CHLORFENVINPHOS = phosphate de 2-chloro-1-(2,4-dichlorophényl)-éthényl-diéthyle;
LINDANE = 1$\alpha$, 2,$\alpha$ 3$\beta$, 4$\alpha$, 5$\alpha$, 6$\beta$-hexachlorocyclohexane;
TIFATOL = N-(2,3-dihydro-3-méthyl-1,3-thiazol-2-ylidène)-2,4-xylidine;
PHOXIM = 0-($\alpha$ -cyanobenzylidèneamino)-0'-0"-diéthylthiophosphate;
FLUMETHRIN = ester $\alpha$-cyano-4-fluoro-3-phénoxy-benzylique de l'acide 3-[2-(4-chlorophényl)-2-chlorovinyl]-2,2-diméthylcyclopropanecarboxylique;
CYROMAZIN = 2-cyclopropylamino-4,6-diamino-s-triazine;
CYHALOTHRIN = (D,L)- $\alpha$-cyano-3-phénoxybenzyl(Z)-(1DL,3DL) -(2-chloro-3,3,3-trifluoropropényl)-2,2-diméthylcyclopropanecarboxylate;
DELTAMETHRIN = (L)- $\alpha$-cyano-3-phénoxybenzyl-(1D,3D)-3-(2,2-dibromovinyl)-2,2-diméthylcyclopropanecarboxylate;
FENVALERAT = (DL)-$\alpha$-cyano-3-phénoxybenzyl-(DL)-2-(4-chlorophényl)-3-méthylbutyrate;
PERMETHRIN = 3-phénoxybenzyl-(1DL)-cis,trans-3-(2,2-dichlorovinyl)-2,2-diméthylcyclopropanecarboxylate;
CYPERMETHRIN = (DL)-$\alpha$-cyano-3-phénoxybenzyl-(1DL)-cis, trans-3-(2,2-dichlorovinyl)-2,2-diméthylcyclopropanecarboxylate;
FAMPHUR = 0-[4-(diméthylsulfamoyl)-phényl]-0,0-diméthylphosphorothioate;
FENTHION = 0,0-diméthyl-0-4-méthylthio-m-tolylphosphorothioate;
PHOSMET = 0,0-diméthyl-5-phtalimidométhylphosphorodithioate;
TETRACHLORVINPHOS = [(Z)-2-chloro-1-(2,4,5-trichlorophényl)-vinyl]-diméthylphosphate;
PROMACYL = 5-méthyl-m-cuménylbutyryl(méthyl)carbamate; et
DIFLUBENZURON = 1-(4-chlorophényl)-3-(2,6-difluoro-benzoyl)-urée.

7. Formulation "pour-on" non aqueuse selon la revendication 6, caractérisée en ce qu'on introduit comme agent actif du Diazinon.

8. Formulation "pour-on" non aqueuse selon la revendication 6, caractérisée en ce qu'on introduit comme agent actif de la Cypermethrin.

9. Formulation "pour-on" liquide, non aqueuse selon la revendication 5, caractérisée en ce qu'elle contient comme agent à activité ectoparasiticide une substance de formule X

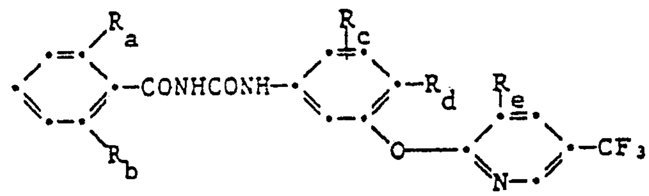

(X)

où

$R_a$ désigne l'hydrogène, un alcoxy en $C_1$-$C_2$, un alkylthio en $C_1$-$C_2$, un alkyle en $C_1$-$C_2$ ou un halogène ;

$R_b$ représente l'hydrogène ou un halogène ;

$R_c$ désigne l'hydrogène ou un halogène ;

$R_d$ représente l'hydrogène, un alkyle en $C_1$-$C_2$ ou un halogène ; et

$R_e$ représente un halogène.

**10.** Formulation selon la revendication 9, caractérisée en ce qu'elle contient comme agent actif une substance choisie parmi les suivantes :

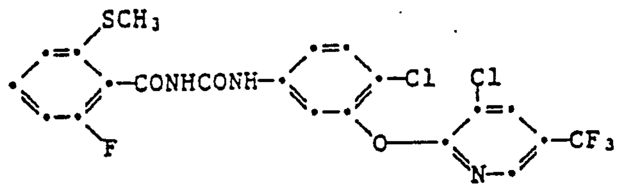

,

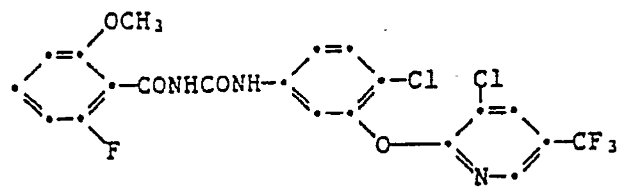

,

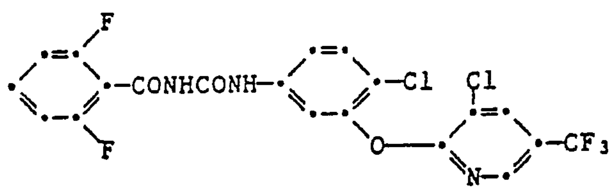

,

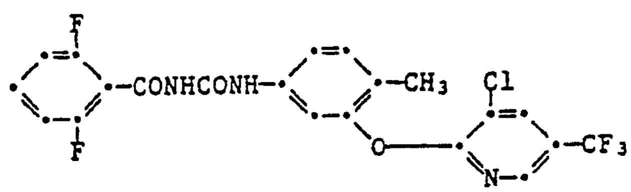

,

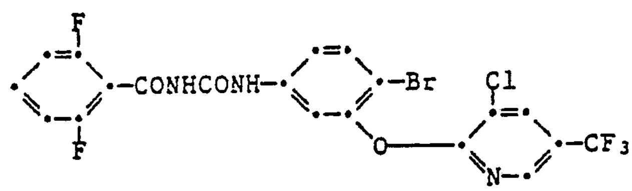

,

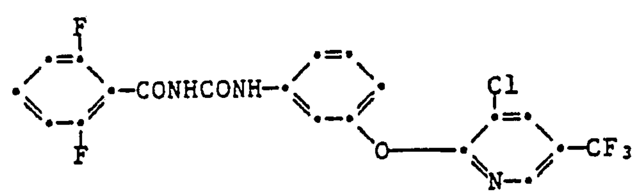

et

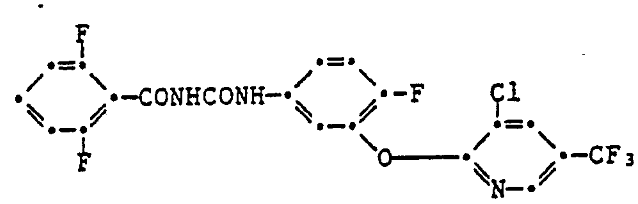

.

**11.** Formulation "pour-on" liquide, non aqueuse selon la revendication 5, caractérisée en ce qu'elle contient comme agent à activité ectoparasiticide une substance de formule XX

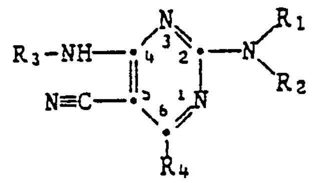

(XX),

où

- $R_1$ représente l'hydrogène, un alkyle en $C_1$ -$C_6$, un alcényle en $C_C$-$C_6$ ou un alcynyle en $C_2$-$C_6$ ;
- $R_2$ désigne l'hydrogène, un alkyle en $C_1$-$C_{10}$ ou un cycloalkyle en $C_3$-$C_6$ ; ou
- $R_1$ et $R_2$ représentent ensemble l'un des restes $-(CH_2)_3-$, $-(CH_2)_4-$ ou $-(CH_2)_5-$ ;
- $R_3$ représente l'hydrogène ou l'un des restes $-CO-R_5$ ou $-SO_2-R_6$ ;
- $R_4$ désigne l'un des restes

  $-NH_2$, $-NH-CO-R_5$, $-NH-SO_2-R_6$ ;

  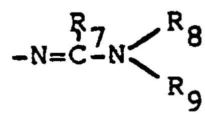

  ou $-NH-CH=N-R_{10}$ ;
- $R_5$ désigne l'hydrogène, un alkyle en $C_1$-$C_{12}$, un cycloalkyle en $c_3$-$c_6$, un alcoxy en $C_1$-$C_6$, un alkyle en $C_1$-$c_3$ perhalogéné ou le reste

  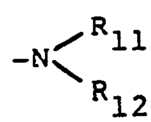

  ;
- $R_6$ est un alkyle en $C_1$-$C_6$ ;
- $R_7$ est l'hydrogène ou un alkyle en $C_1$-$C_6$ ;
- $R_8$ et $R_9$ représentent, indépendamment l'un de l'autre, l'hydrogène ou un alkyle en $C_1$-$C_6$ ou ensemble l'un des restes $-(CH_2)_3-$, $-(CH_2)_4-$ ou $-(CH_2)_5-$ ;
- $R_{10}$ représente l'un des restes $-SO_2-R_{13}$ ou

  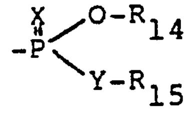

  ;
- $R_{11}$ et $R_{12}$ représentent, indépendamment l'un de l'autre, l'hydrogène ou un alkyle en $C_1$-$C_6$ ou ensemble l'un des restes $-(CH_2)_3-$, $-(CH_2)_4-$ ou $-(CH_2)_5-$ ;
- $R_{13}$ est un alkyle en $C_1$-$C_{10}$, un alkyle en $C_1$-$C_{10}$ substitué par jusqu'à 10 atomes d'halogène ou un cycloalkyle en $C_3$-$C_6$ ;
- $R_{14}$ et $R_{15}$ sont, indépendamment l'un de l'autre, un alkyle en $C_1$-$C_{10}$ ;
- X et Y représentent, indépendamment l'un de l'autre, de l'oxygène ou du soufre,

  ainsi que leurs sels.

**12.** Procédé pour la préparation d'une formulation "pour-on" liquide, non aqueuse selon la revendication 1, caractérisé en ce qu'on dissout ou émulsifie ou met en suspension, éventuellement avec addition d'agents de surface, l'agent actif ectoparasiticide dans de l'alcool diacétonique et, si nécessaire, on

introduit ou on ajoute au mélange le reste des ingrédients auxiliaires.

**13.** Utilisation d'alcool diacétonique pour la préparation de formulations "pour-on" liquides, non aqueuses.

**14.** Formulations "pour-on" non aqueuses selon la revendication 1 pour la lutte contre les ectoparasites sur les animaux utiles.

**Revendications pour les Etats contractants suivants : ES, GR**

**1.** Procédé pour la préparation d'une formulation "pour-on" liquide, non aqueuse d'agents actifs ectoparasiticides destinée aux animaux utiles, qui contient, outre l'agent actif ou le mélange d'agents actifs, en tant que constituant principal de l'alcool diacétonique et éventuellement en tant que constituant secondaire un ou plusieurs autres adjuvants de formulation convenables, caractérisé en ce qu'on dissout ou émulsifie ou met en suspension, éventuellement avec addition d'agents de surface, l'agent actif ectoparasiticide dans de l'alcool diacétonique et, si nécessaire, on introduit ou on ajoute au mélange le reste des ingrédients auxiliaires.

**2.** Procédé pour la préparation d'une formulation "pour-on" non aqueuse selon la revendication 1, caractérisé en ce que la teneur en alcool diacétonique est égale à plusieurs fois la teneur en d'autres adjuvants de formulation.

**3.** Procédé pour la préparation d'une formulation "pour-on" non aqueuse selon la revendication 2, caractérisé en ce qu'elle comporte 0,1 à 60% en poids de l'agent actif, 40 à 99,9% en poids d'alcool diacétonique et 0 à 20% en poids d'autres adjuvants de formulation.

**4.** Procédé pour la préparation d'une formulation "pour-on" non aqueuse selon la revendication 3, caractérisé en ce qu'on introduit comme autres adjuvants de formulation des cosolvants, dse agents stabilisants, des colorants, des antioxydants, des agents de surface ou des agents de fixation.

**5.** Procédé pour la préparation d'une formulation "pour-on" non aqueuse selon l'une quelconque des revendications 1 à 4, caractérisé en ce qu'on utilise un agent actif ectoparasiticide choisi parmi les composés organophosphorés, les composés organochlorés, les pyréthroïdes, les carbamates, les amidines, les dimiloïdes, les cyanopyrimidines ou les triazines.

**6.** Procédé pour la préparation d'une formulation "pour-on" liquide, non aqueuse selon la revendication 5, caractérisé en ce qu'elle contient en tant qu'agent actif ectoparasiticide une substance choisie parmi les suivantes :
AMITRAZ = N,N-bis-(2,4-xylyliminométhyl)méthylamine;
PROPETAMPHOS = ester d'acide 1-méthyléthyl-(E)-3-[[(éthylamino)méthoxyphosphinothioyl]-oxy]-2-buténique;
CHLORPYRIFOS = thiophosphate de 0,0-diéthyl-0-(3,5,6-trichloro-2-pyridinyle);
DIAZINON = thiophosphate de 0,0-diéthyl-0-[6-méthyl-2-(1-méthyléthyl)-4-pyrimidinyle);
COUMAPHOS = thiophosphate de 0-(3-chloro-4-méthyl-2-oxo-2H-1-benzopyrann-7-yl) -0,0-diéthyle;
MALATHION = [(diméthoxyphosphinothioyl)thio]-succinate de diéthyle;
CHLORFENVINPHOS = phosphate de 2-chloro-1-(2,4-dichlorophényl)-éthényl-diéthyle;
LINDANE = 1$\alpha$, 2$\alpha$, 3$\beta$, 4$\alpha$, 5$\alpha$, 6$\beta$-hexachlorocyclohexane;
TIFATOL = N-(2,3-dihydro-3-méthyl-1,3-thiazol-2-ylidène)-2,4-xylidine;
PHOXIM = 0-( $\alpha$-cyanobenzylidèneamino)-0'-0"-diéthylthiophosphate;
FLUMETHRIN = ester $\alpha$-cyano-4-fluoro-3-phénoxy-benzylique de l'acide 3-[2-(4-chlorophényl)-2-chlorovinyl]-2,2-diméthylcyclopropanecarboxylique;
CYROMAZIN = 2-cyclopropylamino-4,6-diamino-s-triazine;
CYHALOTHRIN = (D,L)- $\alpha$-cyano-3-phénoxybenzyl(Z)-(1DL,3DL) -(2-chloro-3,3,3-trifluoropropényl)-2,2-diméthylcyclopropanecarboxylate;
DELTAMETHRIN = (L)- $\alpha$-cyano-3-phénoxybenzyl-(1D, 3D)-3-(2,2-dibromovinyl)-2,2-diméthylcyclopropanecarboxylate;
FENVALERAT = (DL)-$\alpha$-cyano-3-phénoxybenzyl-(DL)-2-(4-chlorophényl)-3-méthylbutyrate;
PERMETHRIN = 3-phénoxybenzyl-(1DL)-cis,trans-3-(2,2-dichlorovinyl)-2,2-diméthylcyclopropanecarboxylate;

CYPERMETHRIN = (DL)-α-cyano-3-phénoxybenzyl-(IDL)-cis, trans-3-(2,2-dichlorovinyl)-2,2-diméthylcyclopropane-carboxylate;
FAMPHUR = O-[4-(diméthylsulfamoyl)-phényl]-O,O-diméthyl-phosphorothioate;
FENTHION = O,O-diméthyl-O-4-méthylthio-m-tolylphosphoro-thioate;
PHOSMET = O,O-diméthyl-5-phtalimidométhylphosphoro-dithioate;
TETRACHLORVINPHOS = [(Z)-2-chloro-1-(2,4,5-trichloro-phényl)-vinyl]-diméthylphosphate;
PROMACYL = 5-méthyl-m-cuménylbutyryl(méthyl)carbamate; et DIFLUBENZURON = 1-(4-chlorophényl)-3-(2,6-difluoro-benzoyl)-urée.

7. Procédé pour la préparation d'une formulation "pour-on" non aqueuse selon la revendication 6, caractérisé en ce qu'on introduit comme agent actif du Diazinon.

8. Procédé pour la préparation d'une formulation "pour-on" non aqueuse selon la revendication 6, caractérisé en ce qu'on introduit comme agent actif de la Cypermethrin.

9. Procédé pour la préparation d'une formulation "pour-on" liquide, non aqueuse selon la revendication 5, caractérisé en ce qu'elle contient comme agent à activité ectoparasiticide une substance de formule X

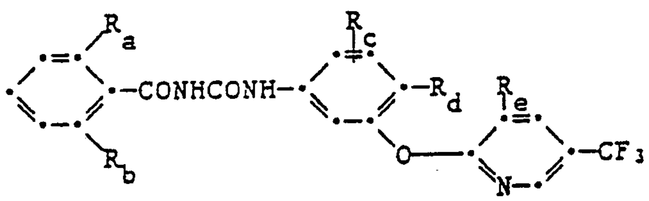

(X)

où

$R_a$ désigne l'hydrogène, un alcoxy en $C_1$-$C_2$, un alkylthio en $C_1$-$C_2$, un alkyle en $C_1$-$C_2$ ou un halogène ;
$R_b$ représente l'hydrogène ou un halogène ;
$R_c$ désigne l'hydrogène ou un halogène ;
$R_d$ représente l'hydrogène, un alkyle en $C_1$-$C_2$ ou un halogène ; et
$R_e$ représente un halogène.

10. Procédé pour la préparation d'une formulation "pour-on" liquide, non aqueuse selon la revendication 9, caractérisé en ce qu'elle contient comme agent actif une substance choisie parmi les suivantes :

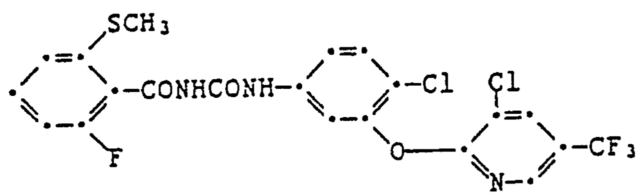 ,

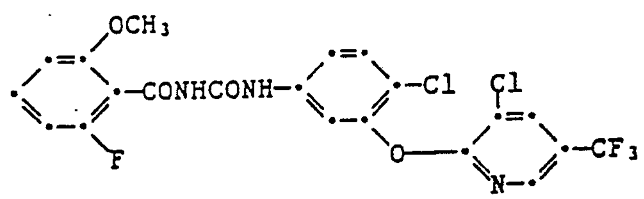 ,

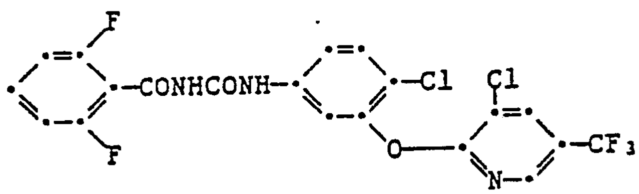 ,

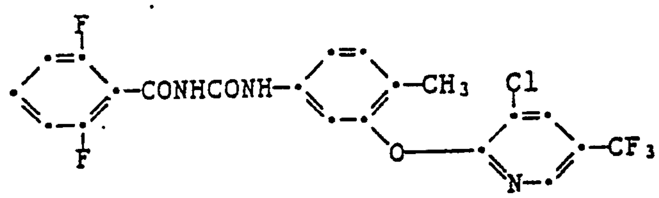 ,

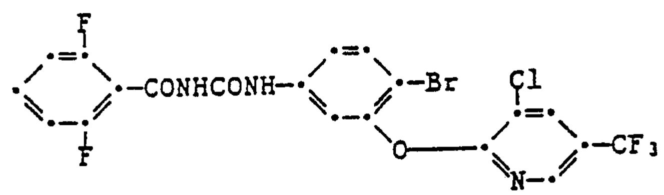 ,

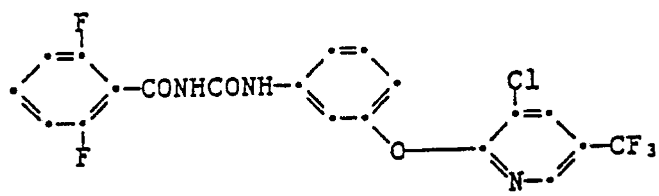

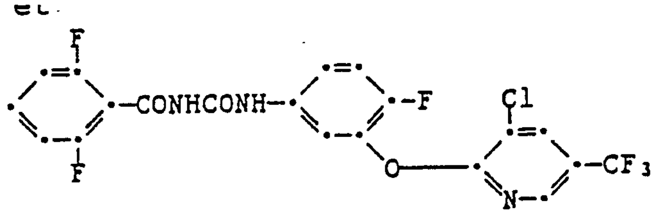

**11.** Procédé pour la préparation d'une formulation "pour-on" liquide, non aqueuse selon la revendication 5, caractérisé en ce qu'elle contient comme agent à activité ectoparasiticide une substance de formule XX

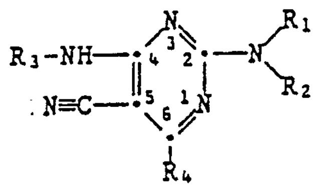

(XX),

où

$R_1$ représente l'hydrogène, un alkyle en $C_1$-$6_6$, un alcényle en $C_c$.$C_6$ ou un alcynyle en $C_2$-$C_6$ ;

$R_2$ désigne l'hydrogène, un alkyle en $C_1$-$C_{10}$ ou un cycloalkyle en $C_3$-$C_6$ ; ou

$R_1$ et $R_2$ représentent ensemble l'un des restes $-(CH_2)_3-$, $-(CH_2)_4-$ ou $-(CH_2)_5-$ ;

$R_3$ représente l'hydrogène ou l'un des restes $-CO-R_5$ ou $-SO_2-R_6$ ;

$R_4$ désigne l'un des restes $-NH_2$, $-NH-CO-R_5$, $-NH-SO_2-R_6$ ;

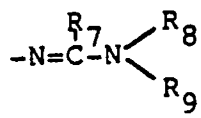

ou $-NH-CH=N-R_{10}$ ;

$R_5$ désigne l'hydrogène, un alkyle en $C_1$-$C_{12}$, un cycloalkyle en $C_3$-$C_6$, un alcoxy en $C_1$-$C_6$, un alkyle en $C_1$-$C_3$ perhalogéné ou le reste

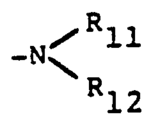

;

$R_6$ est un alkyle en $C_1$-$C_6$ ;

$R_7$ est l'hydrogène ou un alkyle en $C_1$-$C_6$ ;

$R_8$ et $R_9$ représentent, indépendamment l'un de l'autre, l'hydrogène ou un alkyle en $C_1$-$C_6$ ou ensemble l'un des restes $-(CH_2)_3-$, $-(CH_2)_4-$ ou $-(CH_2)_5-$ ;

$R_{10}$ représente l'un des restes $-SO_2-R_{13}$ ou

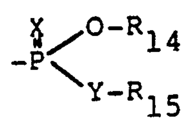

;

$R_{11}$ et $R_{12}$ représentent, indépendamment l'un de l'autre, l'hydrogène ou un alkyle en $C_1$-$C_6$ ou ensemble l'un des restes $-(CH_2)_3-$, $-(CH_2)_4-$ ou $-(CH_2)_5-$ ;

$R_{13}$ est un alkyle en $C_1$-$C_{10}$, un alkyle en $C_1$-$C_{10}$ substitué par jusqu'à 10 atomes d'halogène ou un cycloalkyle en $C_3$-$C_6$ ;

$R_{14}$ et $R_{15}$ sont, indépendamment l'un de l'autre, un alkyle en $C_1$-$C_{10}$ ;

X et Y représentent, indépendamment l'un de l'autre, de l'oxygène ou du soufre,

ainsi que leurs sels.

**12.** Utilisation d'alcool diacétonique pour la préparation de formulations "pour-on" liquides, non aqueuses.